# EUROPÄISCHE PATENTSCHRIFT

(11) **EP 1 503 722 B1**
(45) Veröffentlichungstag und Bekanntmachung des Hinweises auf die Patenterteilung: **18.07.2007**
(21) Anmeldenummer: 03729971.6
(22) Anmeldetag: 02.05.2003
(51) Int. Cl.: A61Q 5/00, A61Q 19/00, A61K 8/81, C08F 226/10, C08F 226/06, C08F 251/00

(54) **KOSMETISCHES MITTEL ENTHALTEND WENIGSTENS EIN WASSERLÖSLICHES COPOLYMER MIT (METH)ACRYLSÄUREAMIDEINHEITEN**
COSMETIC PRODUCT COMPRISING AT LEAST ONE WATER-SOLUBLE COPOLYMER WHICH CONTAINS (METH)ACRYLAMIDE UNITS
PRODUIT COSMETIQUE CONTENANT AU MOINS UN COPOLYMERE AYANT DES UNITES AMIDES D'ACIDE (METH)ACRYLIQUE ET SOLUBLE DANS L'EAU

(30) Priorität: 03.05.2002 DE 10219889; 19.09.2002 DE 10243573
(43) Veröffentlichungstag der Anmeldung: 09.02.2005
(73) Patentinhaber: BASF Aktiengesellschaft, 67056 Ludwigshafen (DE)
(72) Erfinder: NGUYEN-Kim, Son, 69502 Hemsbach (DE); HÖSSEL, Peter, 67105 Schifferstadt (DE); MÜLLER, Gabi, 67071 Ludwigshafen (DE)
(74) Vertreter: Reitstötter - Kinzebach
(86) Internationale Anmeldenummer: PCT/EP2003/004647
(87) Internationale Veröffentlichungsnummer: WO 2003/092640

(56) Entgegenhaltungen:
- EP-A- 1 002 811
- WO-A-01/62809
- WO-A-03/053381
- DE-A- 19 630 977
- US-A- 3 006 900
- US-A- 4 237 253

## Beschreibung

Die vorliegende Erfindung betrifft ein kosmetisches Mittel, welches wenigstens ein wasserlösliches Copolymer enthält, das durch radikalische Copolymerisation von Acrylsäureamid und/oder Methacrylsäureamid und weiteren damit copolymerisierbaren wasserlöslichen α,β-ethylenisch ungesättigten Verbindungen, gegebenenfalls in Gegenwart einer wasserlöslichen polymeren Pfropfgrundlage, erhältlich ist.

Kosmetisch und pharmazeutisch akzeptable wasserlösliche Polymere finden in Kosmetik und Medizin vielfache Anwendung. In Seifen, Cremes und Lotionen beispielsweise dienen sie in der Regel als Formulierungsmittel, z.B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen.

Für die Haarkosmetik werden filmbildende Polymere beispielsweise als Conditioner dazu eingesetzt, um die Trocken- und Nasskämmbarkeit, Anfassgefühl, Glanz und Erscheinungsform zu verbessern sowie dem Haar antistatische Eigenschaften zu verleihen. Bevorzugt werden wasserlösliche Polymere mit polaren, häufig kationischen Funktionalitäten eingesetzt, die eine größere Affinität zur strukturell bedingt negativ geladenen Oberfläche des Haares aufweisen. Struktur und Wirkungsweise verschiedener Haarbehandlungspolymere sind in Cosmetic & Toiletries 103 (1988) 23 beschrieben. Handelsübliche Conditionerpolymere sind z.B. kationische Hydroxyethylcellulose, kationische Polymere auf der Basis von N-Vinylpyrrolidon, z.B. Copolymere aus N-Vinylpyrrolidon und quarterniertem N-Vinylimidazol, Acrylamid und Diallyldimethylammoniumchlorid oder Silicone.

Zur Festigung von Haarfrisuren werden beispielsweise Vinyllactam-Homo- und Copolymere und Carboxylatgruppen-haltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares.

Schwierigkeiten bereitet oft die Bereitstellung von Produkten mit einem komplexen Eigenschaftsprofil. So besteht ein Bedarf an Polymeren für kosmetische Mittel, die zur Bildung im Wesentlichen glatter, klebfreier Filme befähigt sind, die dem Haar und der Haut einen angenehmen Griff verleihen und gleichzeitig eine gute Konditionierwirkung bzw. Festigungswirkung aufweisen. Zudem werden an kosmetische und pharmazeutische Produkte vom Verbraucher zunehmend ästhetische Anforderungen gestellt. So wird bei derartigen Produkten derzeit eine Bevorzugung von klaren, opaquen Formulierungen in Form von Gelen beobachtet.

Die DE-PS-963 057 beschreibt Polymere auf Basis von Vinylimidazolen, die weitere Comonomere einpolymerisiert enthalten können. Als geeignete Comonomere werden u. a. Vinylpyrrolidon, Vinylcaprolactam und Acrylamid genannt.

Die US 3,269,969 beschreibt die Herstellung von Dispergiermitteln durch Wärmebehandlung von (Meth)acrylsäureamidcopolymeren in Gegenwart von Wasser. Dabei wird eine Zunahme des K-Werts bei gleichzeitiger Verbesserung der Wasserlöslichkeit erzielt. Die eingesetzten Copolymere können eine Vielzahl weiterer Monomere eingebaut enthalten, wobei u. a. Vinyllactame und (Meth)acrylsäureamide genannt werden.

Die DE-AS-1 006 151 beschreibt ein Verfahren zur Herstellung von Homo- und Copolymerisaten aus ungesättigten Carbonsäureamiden. Als geeignete Comonomere werden u. a. ungesättigte Carbonsäureamide und Vinyllactame genannt.

Die DE-AS-1 090 079 beschreibt die Verwendung von wässrigen Polymerdispersionen, die Copolymerisate mit hohem Anteil ungesättigter Carbonsäureamide enthalten, als Hilfsmittel zur Papierveredlung.

Ein Einsatz der zuvor genannten Polymere in der Kosmetik wird nicht beschrieben.

Die US 5,478,553 und US 5,632,977 beschreiben Haarfestigerzusammensetzungen, die Mono- oder Copolymere von N-Vinylformamid enthalten. Als geeignetes Comonomer wird u. A. auch Acrylamid genannt.

Aus der US-A-5,334,287 sind Pfropfpolymerisate bekannt, die durch radikalisch initiierte Polymerisation von N-Vinylcarbonsäureamiden, vorzugsweise N-Vinylformamid, und gegebenenfalls anderen Monomeren in Gegenwart von Monosacchariden, Oligosacchariden und Polysacchariden erhältlich sind. Als zusätzliche Monomere werden neben einer Vielzahl weiterer auch Acrylamid und Methacrylamid genannt. Eine Eignung dieser Pfropfcopolymere als Wirkstoff in kosmetischen Formulierungen wird nicht genannt.

Die WO 02/15854 beschreibt die Verwendung von Pfropfcopolymerisaten, die erhältlich sind durch radikalische Pfropfcopolymerisation von mindestens einer offenkettigen N-Vinylamid-Verbindung und gegebenenfalls wenigstens eines weiteren damit copolymerisierbaren Monomeren auf eine polymere Pfropfgrundlage, für kosmetische Anwendungen.

Die GB 1 602 420 beschreibt in den Beispielen 20, 21, 23 und 35 Zusammensetzungen, die Acrylamid oder Methacrylamid, N-Vinylpyrrolidon sowie zusätzlich Dimethyldially-lammoniumbromid einpolymerisiert enthalten.

Die US 3,006,900 beschreibt Methacrylamid/N-Vinylpyrrolidon-Copolymere, jedoch nicht deren Einsatz in der Kosmetik.

Der vorliegenden Erfindung liegt die Aufgabe zugrunde, kosmetische und pharmazeutische Mittel mit guten anwendungstechnischen Eigenschaften zur Verfügung zu stellen. Diese sollen zur Bildung von klebfreien glatten Filmen befähigt sein. Sie sollen insbesondere eine gute Festigungswirkung aufweisen und sich zur Herstellung von Produkten in Form von Gelen eignen.

Überraschenderweise wurde nun gefunden, dass diese Aufgabe durch ein kosmetisches Mittel gelöst wird, das wenigstens ein wasserlösliches Copolymer enthält, welches durch radikalische Copolymerisation von Acrylsäureamid und/oder Methacrylsäureamid und weiteren damit copolymerisierbaren wasserlöslichen α,β-ethylenisch ungesättigten Verbindungen, gegebenenfalls in Gegenwart einer wasserlöslichen polymeren Pfropfgrundlage, erhältlich ist.

Gegenstand der Erfindung ist daher ein kosmetisches oder pharmazeutisches Mittel, enthaltend
A) wenigstens ein wasserlösliches oder wasserdispergierbares Copolymer, das erhältlich ist durch radikalische Copolymerisation von
   a) 5 bis 90 Gew.-%, bezogen auf das Gesamtgewicht der Komponenten a) bis d), Acrylsäureamid und/oder Meth- acrylsäureamid,
   b) 5 bis 85 Gew.-%, bezogen auf das Gesamtgewicht der Komponenten a) bis d), wenigstens einer α,β-ethylenisch ungesättigten amidgruppenhaltigen Verbindung der allgemeinen Formel I 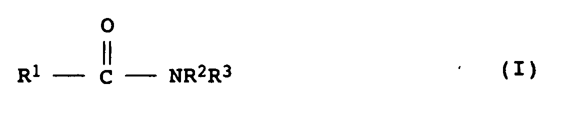 wobei
      R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen, mit der Maßgabe, das einer der Reste R² und R³ von H verschieden ist, oder R² und R³ gemeinsam mit dem Stickstoffatom, an das sie gebunden sind, für einen fünfbis achtgliedrigen Heterocyclus stehen,
      oder R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind für ein Lactam mit 5 bis 8 Ringatomen stehen,
   c) 0 bis 40 Gew.-%, bezogen auf das Gesamtgewicht der Komponenten a) bis d), wenigstens einer von den Komponenten a) und b) verschiedenen, damit copolymerisierbaren ungesättigten wasserlöslichen Verbindung, die ausgewählt ist unter Estern α,β-ethylenisch ungesättigter Mono- und Dicarbonsäuren mit Aminoalkoholen, deren N-Alkyl- und N,N-Dialkylderivaten; Estern von Vinylalkohol mit Monocarbonsäuren; vinyl- und allylsubstituierten heteroaromatischen Verbindungen; Amiden α,β-ethylenisch ungesättigter Mono- und Dicarbonsäuren mit Diaminen, die eine tertiäre und eine primäre oder sekundäre Aminogruppe aufweisen; Polyetheracrylaten und Mischungen davon,
   wobei der Gewichtsmengenanteil der Summe der Komponenten b) und c) wenigstens 5 Gew.-% beträgt,
   gegebenenfalls in Gegenwart von bis zu 25 Gew.-%, bezogen auf das Gesamtgewicht der Komponenten a) bis d), wenigstens einer wasserlöslichen Komponente d), die ausgewählt ist unter
   d1) polyetherhaltigen Verbindungen,
   d2) Polymeren, die mindestens 50 Gew.-% Wiederholungseinheiten aufweisen, die sich von Vinylalkohol ableiten,
   d3) Stärke und Stärkederivaten,
   und Mischungen davon, und
B) wenigstens einen kosmetisch akzeptablen Träger.

Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl(en), n-Decyl(en), n-Undecyl(en), -n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Cycloalkyl steht vorzugsweise für C₅-C₉-Cycloalkyl, wie Cyclopentyl, Cyclohexyl, Cycloheptyl oder Cyclooctyl.

Der Ausdruck Heterocycloalkyl im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR^{a}, COO-M⁺ und NE¹E², bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl; Tetrahydropyranyl, Dioxanyl genannt.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Mittel lassen sich unter Normalbedingungen (20°C) vorteilhaft als Gele formulieren. "Gelförmige Konsistenz" zeigen Mittel, die eine höhere Viskosität als eine Flüssigkeit aufweisen und die selbsttragend sind, d.h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegen vorzugsweise in einem Bereich von größer als 600 bis etwa 60 000 mPas. Vorzugsweise handelt es sich bei den Gelen um Haargele, wobei dies eine Viskosität von vorzugsweise 6000 bis 30000 mPas aufweisen.

Unter wasserlöslichen Monomeren und Polymeren werden im Rahmen der vorliegenden Erfindung Monomere und Polymere verstanden, die sich zu mindestens 1 g/l bei 20°C in Wasser lösen. Unter wasserdispergierbaren Polymeren werden Polymere verstanden, die unter Anwendung von Scherkräften beispielsweise durch Rühren in dispergierbare Partikel zerfallen.

Die erfindungsgemäßen und zur Herstellung der erfindungsgemäßen kosmetischen Mittel eingesetzten Copolymere A) enthalten vorzugsweise keine säuregruppenhaltigen Monomere einpolymerisiert.

Erfolgt die radikalische Copolymerisation der Komponenten a) sowie gegebenenfalls b) und/oder c) in Gegenwart wenigstens einer Verbindung der Komponente d) werden Copolymere A) mit vorteilhaften Eigenschaften erhalten. Dies kann beispielsweise aus einer zumindest teilweisen Pfropfung auf die Komponente d) als Pfropfgrundlage resultieren. Es sind jedoch auch andere Mechanismen als eine Pfropfung vorstellbar. Die Komponente A) umfasst ganz allgemein die Verfahrensprodukte der radikalischen Copolymerisation worunter z.B. reine Pfropfpolymerisate, Mischungen von Pfropfpolymerisaten mit ungepfropften Verbindungen der Komponente d), Homo- und Copolymerisate der Monomeren a) und gegebenenfalls b) und/oder c) sowie beliebige Mischungen verstanden werden. Anteile von ungepfropften Verbindungen der Komponente d) können je nach Verwendungszweck der Copolymere A) von Vorteil sein. Ihnen kann beispielsweise eine Wirkung als Emulgator oder Schutzkolloid zukommen.

Das Copolymer A) enthält 5 bis 90 Gew.-%, vorzugsweise 10 bis 85 Gew.-%, besonders bevorzugt 15 bis 80 Gew.-%, bezogen auf das Gesamtgewicht der Komponenten a) bis d), Acrylsäureamid und/oder Methacrylsäureamid einpolymerisiert.

Nach einer bevorzugten Ausführungsform enthält das Copolymer A) 10 bis 80 Gew.-%, wenigstens einer Verbindung der Komponente b) einpolymerisiert.

Bevorzugt sind die Verbindungen der Komponente b) ausgewählt unter N-Vinyllactamen, N-Vinylamiden gesättigter Monocarbonsäuren, N-Alkyl- und N,N-Dialkylamiden α,β-ethylenisch ungesättigter Monocarbonsäuren und Mischungen davon.

Bevorzugte Monomere b) sind N-Vinyllactame und deren Derivate, die z.B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und N-Vinylcaprolactam eingesetzt.

Als Monomere b) geeignete N-Vinylamide sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Als Monomere b) geeignete N-Alkyl- und N,N-Dialkylamide α,β-ethylenisch ungesättigter Monocarbonsäuren sind beispielsweise N-Methyl(meth)acrylamid, N-Ethyl(meth)acrylamid, N-Propyl(meth)acrylamid, N-tert.-Butyl(meth)acrylamid, N,N-Dimethyl(meth)acrylamid, N,N-Diethyl(meth)acrylamid, etc.

Die zuvor genannten Monomere b) können einzeln und in Form von Mischungen eingesetzt werden.

Nach einer bevorzugten Ausführungsform enthält das Copolymer A) 3 bis 30 Gew.-%, besonders bevorzugt 5 bis 25 Gew.-%, wenigstens einer Verbindung der Komponente c) einpolymerisiert.

Bevorzugt sind die Verbindungen der Komponente c) ausgewählt unter α,β-ethylenisch ungesättigten wasserlöslichen Verbindungen mit nichtionischen, katiogenen und kationischen hydrophilen Gruppen.

Bevorzugt handelt es sich bei den kationogenen und/oder kationischen Gruppen der Komponente c) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z.B. mit Carbonsäuren, wie Milchsäure, oder Mineralsäuren, wie Phosphorsäure, Schwefelsäure und Salzsäure, oder durch Quaternisierung, z.B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat. Eine Protonierung oder Quaternisierung kann im Allgemeinen sowohl vor als auch nach der Polymerisation erfolgen.

Geeignete Verbindungen c) sind z.B. die Ester von α,β-ethylenisch ungesättigten Mono- und Dicarbonsäuren mit Aminoalkoholen, vorzugsweise C₂-C₁₂-Aminoalkoholen. Diese können vorzugsweise am Aminstickstoff C₁-C₈-monoalkyliert oder -dialkyliert sein. Als Säurekomponente dieser Ester eignen sich z.B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind
tert.-Butylaminoethyl(meth)acrylat,
N,N-Dimethylaminomethyl(meth)acrylat,
N,N-Dimethylaminoethyl(meth)acrylat,
N,N-Diethylaminoethyl(meth)acrylat,
N,N-Dimethylaminopropyl(meth)acrylat,
N,N-Diethylaminopropyl(meth)acrylat,
N,N-Dimethylaminocyclohexyl(meth)acrylat etc. Bevorzugt werden
N,N-Dimethylaminoethyl(meth)acrylat und
N,N-Dimethylaminopropyl(meth)acrylat eingesetzt.

Als Monomere c) geeignete Ester von Vinylalkohol mit Monocarbonsäuren sind beispielsweise Vinylformiat, Vinylacetat und Vinylpropionat.

Als Monomere c) geeignete vinyl- und allylsubstituierte heteroaromatische Verbindungen sind beispielsweise N-Vinylimidazol und Derivate davon, wie N-Vinyl-2-methylimidazol etc.

Geeignete Monomere c) sind außerdem Allyamine und Allylammoniumsalze, wie Diallylamin, Diallylmethylamin und Diallyldimethylammoniumchlorid.

Geeignete Monomere c) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z.B. N-[2-(dimethylamino)ethyl]-acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid, N-[4-(dimethylamino)cyclohexyl]methacrylamid etc. Bevorzugt werden N-[3-(dimethylamino)propyl]acrylamid, N-[3-(dimethylamino)propyl]methacrylamid eingesetzt.

Geeignete Monomere c) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20 000 auf. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z.B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere. Bevorzugt als Komponente c) sind Polyetheracrylate der allgemeinen Formel II 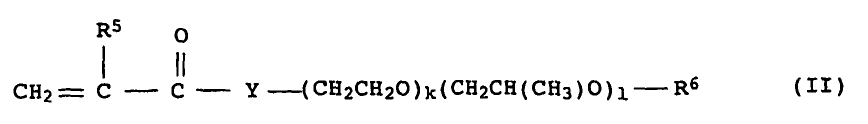 worin
die Reihenfolge der Alkylenoxideinheiten beliebig ist,
- k und 1: unabhängig voneinander für eine ganze Zahl von 0 bis 500 stehen, wobei die Summe aus k und 1 mindestens 5 beträgt,
- R⁵: für Wasserstoff oder C₁-C₈-Alkyl steht, und
- R⁶: für Wasserstoff oder C₁-C₁₈-Alkyl steht,
- Y: für O oder NR⁷ steht, wobei R⁷ für Wasserstoff, C₁-C₈-Alkyl oder C₅-C₈-Cycloalkyl steht.

Bevorzugt steht k für eine ganze Zahl von 1 bis 500, insbesondere 3 bis 250. Bevorzugt steht 1 für eine ganze Zahl von 0 bis 100.

Bevorzugt steht R⁵ für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁶ in der Formel II für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl.

Vorzugsweise steht Y in der Formel II für O oder NH.

Geeignete Polyetheracrylate c) sind z.B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁶-OH hergestellt werden. Die Alkylenoxide können einzeln, alternierend nacheinander oder als Mischung eingesetzt werden. Die Polyetheracrylate c) können allein oder in Mischungen zur Herstellung der erfindungsgemäß eingesetzten Polymere verwendet werden.

Geeignete Polyetheracrylate sind auch Urethan(meth)acrylate mit Alkylenoxidgruppen. Derartige Verbindungen sind in der DE 198 38 851 (Komponente e2)) beschrieben, worauf hier in vollem Umfang Bezug genommen wird.

Die Copolymere A) können gewünschtenfalls wenigstens einen Vernetzer, d.h. eine Verbindung mit 2 oder mehr als 2 ethylenisch ungesättigten Doppelbindungen einpolymerisiert enthalten. Vorzugsweise werden Vernetzer in einer Menge von 0,01 bis 10 Gew.-%, besonders bevorzugt 0,1 bis 3 Gew.-% bezogen auf das Gesamtgewicht der Komponenten a) bis d) eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Besonders bevorzugt als Vernetzer sind Pentaerythrittriallylether, Allylether von Zuckern wie Saccharose, Glucose, Mannose, Divinylbenzol, N,N'-Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin. Ganz besonders bevorzugt sind N,N'-Methylenbisacrylamid, Diallylweinsäurediamid, Diallylphthalat, Diallylharnstoff, Glycoldi(meth)acrylat, Allyl(meth)acrylat sowie Polyallylether.

Nach einer geeigneten Ausführungsform erfolgt die Copolymerisation zur Herstellung der Copolymerisate A) in Gegenwart wenigstens einer Verbindung der Komponente d).

Nach einer bevorzugten Ausführungsform beträgt die Einsatzmenge der Komponente d) 1 bis 25 Gew.-%, besonders bevorzugt 3 bis 20 Gew.-%, bezogen auf das Gesamtgewicht der Komponenten a) bis d).

Die erfindungsgemäß eingesetzten Verbindungen der Komponente d) enthalten im Wesentlichen keine Kohlenstoff-Kohlenstoff-Doppelbindungen. Nach einer geeigneten Ausführungsform enthalten die Verbindungen der Komponente d) keine Siliciumatom-haltigen Gruppen.

Geeignete polyetherhaltige Verbindungen d1) sind im Allgemeinen wasserlösliche oder wasserdispergierbare, nichtionische Polymere, die Polyalkylenglycolgruppen aufweisen. Vorzugsweise beträgt der Anteil an Polyalkylenglycolgruppen mindestens 40 Gew.-%, bezogen auf das Gesamtgewicht der Verbindung d1). Als polyetherhaltige Verbindung d1) können beispielsweise die zuvor genannten Polyalkylenglycole, Polyester auf Basis von Polyalkylenglycolen sowie Polyetherurethane eingesetzt werden.

Je nach Art der zu ihrer Herstellung eingesetzten Monomerbausteine enthalten die polyetherhaltigen Verbindungen d1) folgende Struktureinheiten:
- (CH₂)₂-O-, -(CH₂)₃-O-, -(CH₂)₄-O-, -CH₂-CH(R⁸)-O-, worin

R⁸ für C₁-C₂₄-Alkyl, vorzugsweise C₁-C₄-Alkyl steht.

Die Verbindungen d1) können zusätzlich verbrückende Gruppen aufweisen, die beispielsweise ausgewählt sind unter:
-C(=O)-O-, -O-C(=O)-O-, -C(=O)-NR^{a}-, -O-C(=O)-NR^{a}-, -NR^{b}-(C=O)-NR^{a}-
worin R^{a} und R^{b} unabhängig voneinander für Wasserstoff, C₁-C₃₀-Alkyl, vorzugsweise C₁-C₄-Alkyl oder Cycloalkyl stehen.

Bevorzugt werden als Polyether d1) Polymerisate der allgemeinen Formel III verwendet, mit einem Molekulargewicht >300 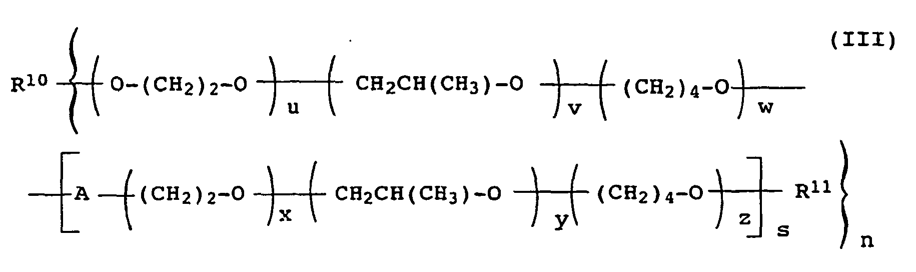 in der die variablen unabhängig voneinander folgende Bedeutung haben:
- R¹⁰: Wasserstoff, C₁-C₂₄-Alkyl, R⁸-C(=O)-, R⁸-NH-C(=O)-, Polyalkoholrest;
- R¹¹: Wasserstoff, C₁-C₂₄-Alkyl, R⁸-C(=O)-, R⁸-NH-C(=O)-;
- R⁸: C₁-C₂₄-Alkyl;
- A: -C(=O)-O, -C(=O)-B-C(=O)-O, -C(=O)-NH-B-NH-C(=O)-O;
- B: -(CH₂)ₜ-, gegebenenfalls substituiertes Cycloalkylen, Heterocycloalkylen oder Arylen;
- n: 1 bis 200, bevorzugt 1 bis 100;
- s: 0 bis 1000, bevorzugt 0 bis 100;
- t: 2 bis 12, bevorzugt 2 bis 6;
- u: 1 bis 1000, bevorzugt 1 bis 500;
- v: 0 bis 1000, bevorzugt 1 bis 500;
- w: 0 bis 1000, bevorzugt 1 bis 500;
- x: 0 bis 1000, bevorzugt 1 bis 500;
- y: 0 bis 1000, bevorzugt 1 bis 500;
- z: 0 bis 1000, bevorzugt 1 bis 500.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden. Bevorzugt werden Polyetherurethane eingesetzt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich größer 300 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen d1) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Nach einer bevorzugten Ausführungsform wird zur Herstellung der Copolymere A) eine Komponente d1) eingesetzt, die wenigstens ein Polyetherurethan umfasst.

Geeignete Polyetherurethane sind die Kondensationsprodukte von Polyetherpolyolen, wie Polyetherdiolen, mit Polyisocyanaten, wie Diisocyanaten. Geeignete Polyetherpolyole sind die zuvor genannten Polyalkylenglycole, die beispielsweise durch aus der Polymerisation von cyclischen Ethern, wie Tetrahydrofuran, oder aus der Umsetzung von einem oder mehreren Alkylenoxiden mit einem Startermolekül, das zwei oder mehr aktive Wasserstoffatome aufweist, erhältlich sind.

Geeignete Polyisocyanate sind ausgewählt unter Verbindungen mit 2 bis 5 Isocyanatgruppen, Isocyanatpräpolymeren mit einer mittleren Anzahl von 2 bis 5 Isocyanatgruppen, und Mischungen davon. Dazu zählen z.B. aliphatische, cycloaliphatische und aromatische Di-, Tri- und Polyisocyanate. Geeignete Diisocyanate sind z.B. Tetramethylendiisocyanat, Hexamethylendiisocyanat, 2,3,3-Trimethylhexamethylendiisocyanat, 1,4-Cyclohexylendiisocyanat, Isophorondiisocyanat, 1,4-Phenylendiisocyanat, 2,4- und 2,6-Toluylendiisocyanat und deren Isomerengemische (z.B. 80 % 2,4- und 20 % 2,6-Isomer), 1,5-Naphthylendiisocyanat, 2,4- und 4,4'-Diphenylmethandiisocyanat. Ein geeignetes Triisocyanat ist z.B. Triphenylmethan-4,4',4"-triisocyanat. Weiterhin geeignet sind Isocyanatpräpolymere und Polyisocyanate, die durch Addition der zuvor genannten Isocyanate an polyfunktionelle hydroxyl- oder amingruppenhaltige Verbindungen erhältlich sind. Weiterhin geeignet sind Polyisocyanate, die durch Biuret- oder Isocyanuratbildung entstehen. Bevorzugt werden Hexamethylendiisocyanat, trimerisiertes Hexamethylendiisocyanat, Isophorondiisocyanat, 2,4-Toluylendiisocyanat, 2,6-Toluylendiisocyanat, und Mischungen davon, eingesetzt.

Als Propfgrundlage eignen sich vorzugsweise weiterhin Polymerisate d2), die mindestens 50 Gew.-% an Vinylalkoholeinheiten besitzen. Bevorzugt enthalten diese Polymerisate mindestens 70 Gew.-%, ganz besonders bevorzugt 80 Gew.-% Polyvinylalkoholeinheiten. Solche Polymerisate werden überlicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters zur Synthese der Pfropfgrundlage d2) kommen beispielsweise N-Vinylcaprolactam, N-Vinylpyrrolidon, N-Vinylimidazol, N-Vinyl-2-methylimidazol, N-Vinyl-4-methylimidazol, 3-Methyl-l-vinylimidazoliumchlorid, 3-Methyl-1-vinylimidazoliummethylsulfat, Diallylammoniumchlorid, Styrol, Alkylstyrole in Frage.

Weitere geeignete Comonomere zur Herstellung der Pfropfgrundlage d2) sind beispielsweise sind monoethylenisch ungesättigten C₃-C₆-Carbonsäuren wie z.B. Acrylsäure, Methacrylsäure, Crotonsäure, Fumarsäure, sowie deren Ester, Amide und Nitrile wie z.B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäureiethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z.B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminothylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Bevorzugte Propfgrundlagen d2) sind Polymerisate, die durch Homopolymerisation von Vinylacetat und anschließender zumindest teilweiser Hydrolyse, Alkoholyse oder Aminolyse hergestellt werden.

Die Herstellung der Pfropfgrundlage d2) erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200°C, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Zur Herstellung der Pfropfgrundlage d2) werden die Estergruppen der ursprünglichen Monomere und gegebenenfalls weiterer Monomere nach der Polymerisation durch Hydrolyse, Alkoholyse oder Aminolyse zumindest teilweise gespalten. Im nachfolgenden wird dieser Verfahrensschritt allgemein als Verseifung bezeichnet. Die Verseifung erfolgt in an sich bekannter Weise durch Zugabe einer Base oder Säure, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base bzw. Säure, von der Verseifungstemperatur, der Verseifungszeit und dem Wassergehalt der Lösung.

Besonders bevorzugte Propfgrundlagen d2) sind Polymerisate, die durch Homopolymerisation von Vinylacetat und anschließender zumindest teilweiser Verseifung hergestellt werden. Solche Polyvinylalkoholeinheiten enthaltenden Polymere sind unter dem Namen Mowiol® erhältlich.

Bevorzugt werden als Komponente d) Stärke und/oder Stärkederivate d3) eingesetzt. Dazu zählen Substanzen, die Saccharid-Strukturen enthalten. Solche natürlichen Substanzen sind beispielsweise Saccharide pflanzlicher oder tierischer Herkunft oder Produkte, die durch Metabolisierung durch Mikroorganismen entstanden sind, sowie deren Abbauprodukte. Geeignete Pfropfgrundlagen d3) sind beispielsweise Oligosaccharide, Polysaccharide, oxidativ, enzymatisch oder hydrolytisch abgebaute Polysaccharide, oxidativ hydrolytisch abgebaute oder oxidativ enzymatisch abgebaute Polysaccharide, chemisch modifizierte Oligo- oder Polysaccharide und Mischungen davon. Bevorzugte Produkte sind die in US 5,334,287 in Spalte 4, Zeile 20 bis Spalte 5, Zeile 45 genannten Verbindungen.

Geeignete kommerziell erhältliche Produkte sind die C-Pur® und C-Dry®-Marken der Fa. Cerestar.

Gewünschtenfalls können Gemische von Verbindungen der Komponente d) eingesetzt werden. Vorteilhaft sind z.B. Gemische, die wenigstens eine Verbindung d2) und wenigstens eine Verbindung d3) enthalten.

Besonders bevorzugt ist das Copolymerisat A) erhältlich durch radikalische Polymerisation von
a) 20 bis 40 Gew.-% Methacrylsäureamid,
b) 40 bis 70 Gew.-% Vinylpyrrolidon,
in Gegenwart von 1 bis 20 Gew.-% Polymeren d2) und/oder Stärke und Stärkederivaten d3).

Des weiteren besonders bevorzugt ist das Copolymerisat A) erhältlich durch radikalische Polymerisation von
a) 30 bis 40 Gew.-% Methacrylsäureamid,
b) 20 bis 60 Gew.-% Vinylpyrrolidon und
   1 bis 20 Gew.-% vinylcaprolactam.

Das erfindungsgemäße und in den erfindungsgemäßen Mitteln eingesetzte Copolymer A) ist in einer zweiten Ausführungsform vorzugsweise erhältlich durch radikalische Copolymerisation von
a) 5 bis 50 Gew.-%, bezogen auf das Gesamtgewicht der Komponenten a) bis d), Methacrylsäureamid,
b) 40 bis 85 Gew.-%, bezogen auf das Gesamtgewicht der Komponenten a) bis d), wenigstens einer Verbindung, ausgewählt unter Vinylpyrrolidon, Vinylcaprolactam, N,N-Dimethylacrylamid und Mischungen davon,
c) 0,2 bis 20 Gew.-%, bezogen auf das Gesamtgewicht der Komponenten a) bis d) wenigstens einer von a) und b) verschiedenen, damit copolymerisierbaren ungesättigten wasserlöslichen Verbindung, die ausgewählt ist unter Vinylimidazol und Derivaten davon, Polyetheracrylaten und Mischungen davon,
gegebenenfalls in Gegenwart von bis zu 10 Gew.-%, bezogen auf das Gesamtgewicht der Komponenten a) bis d), Polymeren d2), die sich von Vinylalkohol ableiten, und gegebenenfalls in Gegenwart von bis zu 1 Gew.-%, bezogen auf das Gesamtgewicht der Komponenten a) bis d), wenigstens eines Vernetzers.

Des Weiteren bevorzugt ist das Copolymerisat A) erhältlich durch radikalische Polymerisation von
a) 7 bis 45 Gew.-% Methacrylsäureamid,
b) 50 bis 80 Gew.-% wenigstens einer Verbindung ausgewählt unter Vinylpyrrolidon, Vinylcaprolactam, N,N-Dimethylacrylamid und Mischungen davon,
c) 0,3 bis 10 Gew.-% wenigstens einer Verbindung, die ausgewählt ist unter Vinylimidazol und Derivaten davon, Polyetheracrylaten und Mischungen davon,
in Gegenwart von 0,1 bis 10 Gew.-% Polymeren d2), die sich von Vinylalkohol ableiten.

Besonders bevorzugt ist das Copolymerisat A) erhältlich durch radikalische Polymerisation von
a) 10 bis 45 Gew.-%, vorzugsweise 10 bis 43 Gew.-%, Methacrylsäureamid,
b) 50 bis 80 Gew.-% Vinylpyrrolidon und Vinylcaprolactam und
c) 0,3 bis 10 Gew.-% Vinylimidazol und/oder eines Derivats davon.

Des Weiteren bevorzugt ist das Copolymerisat A) erhältlich durch radikalische Polymerisation von
a) 10 bis 45 Gew.-%, vorzugsweise 20 bis 45 Gew.-%, besonders bevorzugt 20 bis 43 Gew.-%, und insbesondere 30 bis 40 Gew.-%, Methacrylsäureamid,
b) 50 bis 80 Gew.-%, vorzugsweise 55 bis 70 Gew.-%, Vinylpyrrolidon und
c) 0,5 bis 5 Gew.-%, vorzugsweise 1 bis 4 Gew.-%, Vinylimidazol.

Die Herstellung der Copolymere A) erfolgt nach üblichen, dem Fachmann bekannten Verfahren, vorzugsweise durch Lösungs-Polymerisation.

Die Polymerisationstemperaturen liegen vorzugsweise in einem Bereich von etwa 30 bis 120°C, besonders bevorzugt 40 bis 100°C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren gegebenenfalls in Gegenwart der Komponente d) sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Die Polymerisation kann auch durch Einwirkung von ultravioletter Strahlung, gegebenenfalls in Gegenwart von W-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin und Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Die verwendeten Mengen an Initiator bzw. Initiatorgemischen bezogen auf eingesetzte Monomere liegen im Allgemeinen zwischen 0,01 und 10 Gew.-%, vorzugsweise zwischen 0,1 und 5 Gew.-%.

Die Polymerisation kann beispielsweise in Substanz erfolgen. Bei der Polymerisation in Substanz unter Verwendung einer Pfopfgrundlage d) kann diese in mindestens einem Monomer und eventuell weiteren Comonomeren gelöst und nach Zugabe eines Polymerisationsinitiators kann die Mischung auspolymerisiert werden. Die Polymerisation kann auch halbkontinuierlich durchgeführt werden, indem man zunächst einen Teil, z.B. 10 % des zu polymerisierenden Gemisches aus der Pfropfgrundlage d), mindestens einem Monomeren der Gruppe a), eventuell weiteren Comonomeren und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die Pfopfgrundlage d) in einem Reaktor vorlegt, auf die Polymerisations-temperatur erwärmt und mindestens ein Monomer der Gruppe a), eventuell weiteren Comonomeren und Polymerisationsinitiator entweder auf einmal, absatzweise oder vorzugsweise kontinuierlich zufügt und polymerisiert.

Bevorzugt ist die Polymerisation in einem Lösemittel. Geeignete Lösemittel sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Besonders bevorzugt ist die Polymerisation in Wasser oder einem Wasser/Alkohol-Gemisch, beispielseweise in einem Wasser/Ethanol-Gemisch. Das Verhältnis von Alkohol zu Wasser liegt in solchen Gemischen bevorzugt in einem Bereich von 1:1 bis 1:7 Vol.-%.

Zur Einstellung des Molekulargewichts kann die Polymerisation in Gegenwart wenigstens eines Reglers erfolgen. Als Regler können die üblichen, dem Fachmann bekannten Verbindungen, wie z.B. Schwefelverbindungen, z.B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Bevorzugt werden siliconfreie Regler eingesetzt.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Die Temperatur bei der Haupt- und der Nachpolymerisation beträgt vorzugsweise höchstens 90 °C.

Weiterhin führt man zur Erzielung möglichst reiner Polymere mit geringem Restmonomergehalt die Polymerisation vorzugsweise bei einem pH-Wert im Bereich von 6 bis 8, besonders bevorzugt von 6,4 bis 7,4, durch, um unter den Polymerisationsbedingungen gegebenenfalls entstehenden Ammoniak, der gegebenenfalls mit Monomeren zu unerwünschten Nebenprodukten reakieren kann, zu entfernen. Die Einstellung des pH-Wertes erfolgt durch Zugabe einer geeigneten Säure, wie Milchsäure.

Produkte mit besonders hoher Reinheit und entsprechend vorteilhaften Eigenschaften für einen Einsatz in der Kosmetik können erzielt werden, wenn das Reaktionsprodukt nach der Polymerisation, gegebenenfalls vor und/oder nach einer Nachpolymerisation, einer Wasserdampfdestillation bzw. einem Strippen mit Wasserdampf unterzogen wird. Auch diese Behandlung mit Wasserdampf dient im Wesentlichen der Entfernung von Ammoniak und weiterer unerwünschter, mit Wasserdampf entfernbarer Nebenprodukte aus dem Reaktionsgemisch. Vorzugsweise erfolgt die Wasserdampf-Behandlung zumindest zwischen Haupt- und Nachpolymerisation. Der pH-Wert des Polymerisationsprodukts wird vorzugsweise vor der Wasserdampf-Behandlung auf einen Wert von höchstens 6 eingestellt. Die Temperatur des eingesetzten Wasserdampfs und der behandelten Polymerlösung beträgt vorzugsweise mindestens 90 °C.

Copolymere A), die Basengruppen enthalten, können teilweise oder vollständig neutralisiert werden. Polymere mit Amingruppen können auch durch Quaternisierungsmittel, z.B. mit Alkylierungsmitteln, wie C₁-C₄-Alkylhalogeniden oder -sulfaten in kationische Gruppen überführt werden. Beispiele solcher Alkylierungsmittel sind Ethylchlorid, Ethylbromid, Methylchlorid, Methylbromid, Dimethylsulfat und Diethylsulfat. In aller Regel weisen die erhaltenen Salze der Polymere eine bessere Wasserlöslichkeit oder Dispergierbarkeit in Wasser auf als die nicht neutralisierten oder quaternisierten Polymere.

Wird bei der Herstellung der Polymere ein organisches Lösungsmittel eingesetzt, so kann dieses durch übliche, dem Fachmann bekannte Verfahren, z.B. durch Destillation bei vermindertem Druck, entfernt werden.

Die Polymerlösungen können durch verschiedene Trocknungsverfahren, wie z.B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

Gegenstand der Erfindung sind auch die Copolymere A).

Der kosmetisch akzeptable Träger B) ist vorzugsweise ausgewählt unter
i) Wasser,
ii) wassermischbaren organischen Lösungsmitteln, vorzugsweise C₁-C₄-Alkanolen,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen
und Mischungen davon.

Die erfindungsgemäßen Mittel weisen z.B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z.B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z.B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z.B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z.B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319-355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, dermatologische oder haarkosmetische Mittel handeln.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer A, wenigstens einen wie vorstehend definierten Träger B und wenigstens einen, von Copolymer A verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien, Weichmachern. Geeignete Verdicker sind z. B. die Aculyn®-Marken der Fa. Rohm und Haas, wie Aculyn®22 (Copolymerisat aus Acrylaten und Methacrylsäureethoxilaten mit Stearylrest (20 EO-Einheiten)) und Aculyn®28 (Copolymerisat aus Acrylaten und Methacrylsäureethoxilaten mit Behenylrest (25 EO-Einheiten)).

Geeignete kosmetisch und/oder dermatologisch aktive Wirkstoffe sind z.B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z.B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z.B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z.B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind z.B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon.

Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z.B. 2-Ethyl-1,3-hexandiol, N,N-Diethyl-m-toluamid etc. Geeignete hyperemisierend wirkende Stoffe, welche die Durchblutung der Haut anregen, sind z.B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z.B. Salicylsäure, Kalziumthioglykolat, Thioglykolsäure und ihre Salze, Schwefel, etc. Geeignete Antischuppen-Wirkstoffe sind z.B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z.B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables von Verbindungen der Komponente A) verschiedenes Polymer enthalten. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z.B. Luviset PUR® der Fa. BASF, und Polyharnstoffe. Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luviflex® Soft und Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z.B. Luviset® Marken), Maleinsäureanhydridcopolymere, gegebenenfalls mit Alkohol umgesetzt, anionische Polysiloxane, z.B. carboxyfunktionelle, t-Butylacrylat, Methacrylsäure (z.B. Luviskol® VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z.B. Guarpolymere, wie die Jaguar®-Marken der Fa. Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z.B. Luviskol® Plus (BASF), oder Polyvinylpyrrolidon und deren Copolymere, insbesondere mit Vinylestern, wie Vinylacetat, z.B. Luviskol® VA 37 (BASF); Polyamide, z.B. auf Basis von Itaconsäure und aliphatischen Diaminen, wie sie z.B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z.B. Polyethersiloxane, wie Tegopren® (Fa. Goldschmidt) oder Belsil® (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z.B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise.

Nach einer ersten bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Besonders bevorzugt handelt es sich um Hautpflegemittel, Intimpflegemittel, Fußpflegemittel, Lichtschutzmittel, Repellents, Rasiermittel, Haarentfernungsmittel, Antiaknemittel, Make-ups, Mascara, Lippenstifte, Lidschatten, Kajalstifte, Eyeliner, Rouges und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere A) zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Copolymer A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Copolymere A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z.B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polymeren A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin und azetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z.B. Verbesserung des Anfassgefühls, des Spreitverhaltens, der Wasserresistenz und/oder der Bindung von Wirk- und Hilfsstoffen, wie Pigementen, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben dem Copolymer A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa 4 und 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glycerin oder Polyglycerin; Kondensaten von Ethylenoxid und Propylenglykolen; oxypropylenierten/oxyethylierten C₁₂-C₁₈-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den Polymeren A) auch Wachse verwendet werden, wie z.B. Carnaubawachs, Candilillawachs, Bienenwachs, mikrokristallines Wachs, Ozokeritwachs und Ca-, Mg- und Al-Oleate, -Myristate, -Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75°C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Polymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z.B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z.B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guarhydroxypropyltrimethylammoniumchlorid (INCI: Hydroxylpropyl Guar Hydroxypropyltrimonium Chloride), Copolymere aus N-Vinylpyrrolidon und quaternisiertem N-Vinylimidazol (Polyquaterinium-16, -44, -46), Copolymere aus N-Vinylpyrrolidon/Dimethylaminoethylmethacrylat, quaternisiert mit Diethylsulfat (Polyquaternium-11) und andere.

Weiterhin können die Duschgel-/Shampoo-Formulierungen Verdicker, wie z.B. Kochsalz, PEG-55, Propylene Glykol Oleate, PEG-120 Methyl Glucose Dioleate und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays oder Haarschaums vor. Haarsprays umfassen dabei sowohl Aerosolsprays als auch Pumpsprays ohne Treibgas. Haarschäume umfassen sowohl Aerosolschäume wie auch Pumpschäume ohne Treibgas.

Bevorzugte Haarbehandlungsmittel liegen in Form eines Gels vor. Ein solches Haarbehandlungsmittel enthält beispielsweise:
a) 0,1 bis 20 Gew.-%, bevorzugt 1 bis 10 Gew.-%, mindestens eines Polymers A), wie zuvor definiert,
b) 0 bis 40 Gew.-% wenigstens eines Trägers (Lösungsmittels), der ausgewählt ist unter C₂-C₅-Alkoholen, insbesondere Ethanol,
c) 0,01 bis 5 Gew.-%, bevorzugt 0,2 bis 3 Gew.-%, wenigstens eines Verdickers,
d) 0 bis 50 Gew.-% eines Treibmittels,
e) 0 bis 10 Gew.-%, bevorzugt 0,1 bis 3 Gew.-%, mindestens eines von a) verschiedenen Festigerpolymers, vorzugsweise eines wasserlöslichen nichtionischen Polymers,
f) 0 bis 1 Gew.-% wenigstens eines Rückfetters, vorzugsweise ausgewählt unter Glycerin und Glycerinderivaten,
g) 0 bis 30 Gew.-% weiterer Wirk- und/oder Hilfsstoffe, z.B. wenigstens eine Siliconverbindung,
h) Wasser ad 100 Gew.-%.

Die Haarbehandlungsmittel können weiterhin in Form von Haarsprays oder Haarschäumen vorliegen. Haarsprays und Haarschäume umfassen vorzugsweise überwiegend oder ausschließlich wasserlösliche oder wasserdispergierbare Komponenten. Sind die in den erfindungsgemä-ßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Bevorzugte Haarbehandlungsmittel liegen in Form einer wässrigen Dispersion oder in Form einer alkoholischen oder wässrig-alkoholischen Lösung vor. Beispiele geeigneter Alkohole sind Ethanol, Propanol, Isopropanol und Mischungen davon.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polymere A) können auch in Kombination mit anderen Haarpolymeren in den Mitteln zur Anwendung kommen. Geeignete Polymere sind die zuvor beschriebenen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel in Form eines Haarsprays oder Haarschaums enthält:
a) 0,5 bis 20 Gew.-%, bevorzugt 1 bis 10 Gew.-%, mindestens eines Polymers A), wie zuvor definiert,
b) 50 bis 99,5 Gew.-%, bevorzugt 55 bis 99 Gew.-%, eines Trägers (Lösungsmittels), ausgewählt unter Wasser und wassermischbaren Lösungsmitteln, bevorzugt C₂-C₅-Alkoholen, insbesondere Ethanol, und Mischungen davon,
c) 0 bis 70 Gew.-%, bevorzugt 0,1 bis 50 Gew.-%, eines Treibmittels, vorzugsweise ausgewählt unter Dimethylether und Alkanen, wie z. B. Propan/Butan-Gemischen,
d) 0 bis 10 Gew.-%, bevorzugt 0,1 bis 10 Gew.-%, mindestens eines von a) verschiedenen Haarpolymers, vorzugsweise eines in Wasser löslichen oder dispergierbaren Polymers,
e) 0 bis 0,5 Gew.-%, bevorzugt 0,001 bis 2 Gew.-%, mindestens einer wasserlöslichen oder wasserdispergierbaren Siliconverbindung,
sowie gegebenenfalls weitere Wirkstoffe und/oder Hilfsstoffe, wie zuvor definiert.

Das erfindungsgemäße Mittel kann als Komponente e) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Die Copolymere A) eignen sich in vorteilhafter Weise als Hilfsmittel in der Pharmazie, bevorzugt als oder in Beschichtungsmittel(n) für die Textil-, Papier-, Druck- und Lederindustrie.

Ein weiterer Gegenstand der vorliegenden Erfindung ist ein Verfahren zur Herstellung eines wie vorstehend definierten Copolymeren A durch radikalische Polymerisation der Monomere a) mit wenigstens einem weiteren Monomer ausgewählt unter den Monomeren b) und c) gegebenenfalls in Gegenwart von bis zu 25 Gew.-%, bezogen auf das Gesamtgewicht der Komponenten a) bis d), einer wasserlöslichen Komponente d), dadurch gekennzeichnet, dass man die Polymerisation in einem wässrigen Lösungsmittel durchführt. Die obigen Ausführungen zu den bevorzugten Ausgestaltungen der Polymerisation zur Herstellung des erfindungsgemäßen Copolymers A gelten hier entsprechend.

Die Erfindung wird anhand der folgenden nicht einschränkenden Beispiele näher erläutert.

### Beispiele

Allgemeine Herstellungsvorschrift für Beispiele 1 bis 50 und Vergleichsbeispiele A bis D: Lösungspolymerisation (Beispiel 10)

| | | |
|---|---|---|
| Zulauf 1: | Monomerengemisch aus: | |
| | 240 g | (50 %ige wässrige Lösung) Acrylamid und |
| | 533,4 g | (15 %ige wässrige Lösung) Methacrylamid |
| | | |
| Zulauf 2: | Monomerengemisch aus: | |
| | 120 g | Vinylpyrrolidon und |
| | 80 g | Vinylcaprolactam |
| | | |
| Zulauf 3: | Initiatorlösung aus: | |
| | 4 g | Wako V 50 [2,2'-Azobis(2-amidinopropan)-dihydrochlorid] und |
| | 180 g | Wasser |
| | | |
| Zulauf 4: | Initiatorlösung aus: | |
| | 2 g | Wako V 50 [2,2'-Azobis(2-amidinopropan)-dihydrochlorid] und |
| | 90 g | Wasser |
| | | |
| Zulauf 5: | 1 g | 90%ige Milchsäure in 9 g Wasser |

In einer Rührapparatur mit Rückflusskühler, Innenthermometer und vier separaten Zulaufvorrichtungen wurden 10 % von Zulauf 1, 20 % von Zulauf 2 und 10 % von Zulauf 3 in 490 g Wasser vorgelegt und die Mischung unter Rühren auf ca. 60°C aufgeheizt. Nach dem Anpolymerisieren, erkennbar an einer beginnenden Viskositätserhöhung, wurde bei 65°C der Rest von Zulauf 1 innerhalb von drei Stunden, der Rest von Zulauf 2 innerhalb von 1,5 Stunden und der Rest von Zulauf 3 innerhalb von vier Stunden zugegeben. In einer alternativen Ausführungsform wurde der pH-Wert der Reaktionslösung mittels Zulauf 5 auf 6,4 bis 7,4 eingestellt. Nach dem Ende der Zugabe wurde noch zwei Stunden bei dieser Temperatur nachpolymerisiert. Anschließend wurde zur Nachpolymerisation der Zulauf 4 innerhalb von 30 Minuten bei 65°C zugegeben und nach dem Ende der Zugabe wurde noch ca. zwei Stunden bei dieser Temperatur und weitere zwei Stunden bei einer Temperatur von 80 bis 90°C nachpolymerisiert. In einer alternativen Ausführungsform wurde das Reaktionsgemisch anschließend noch 1 h mit Wasserdampf bei einem pH-Wert von etwa 6 behandelt. Man erhält eine ca. 30 %-wässrige Mikrodispersion. Zum Stabilisieren wird die Lösung mit 100 ppm an Euxyl®K 100 der Fa. Schülke & Mayr (5-Chlor-2-methyl-3-(2H)-isothiazolon / 2-Methyl-3-(2H)-isothiazolon / Benzylalkohol) versetzt. Beim Einsatz von Wasser/Ethanol-Gemischen kann auf den Einsatz eines Stabilisators verzichtet werden.

Pulverförmige Produkte können durch Sprühtrocknen oder Gefriertrocknen erhalten werden. In Analogie wurden alle Produkte in der folgenden Liste polymerisiert.

**Tabelle 1:**

| Bsp.-Nr. | AM | MAM | VP | VCap | VFA | DMAA | 350-MA | Q-DMA EMA | C-Dry MD193 4 | PVOH |
|---|---|---|---|---|---|---|---|---|---|---|
| A | - | - | - | - | - | - | - | - | - | - |
| B | 100 | - | - | - | - | - | - | - | - | - |
| C | - | - | - | - | 100 | - | - | - | - | - |
| D | - | - | 100 | - | - | - | - | - | - | - |
| 1 | 80 | - | 20 | - | - | - | - | - | - | - |
| 2 | 80 | - | - | - | - | 20 | - | - | - | - |
| 3 | 50 | 30 | - | - | - | - | 20 | - | - | - |
| 4 | 70 | - | 30 | - | - | - | - | - | - | - |
| 5 | 70 | - | - | - | - | 30 | - | - | - | - |
| 6 | 60 | - | 40 | - | - | - | - | - | - | - |
| 7 | 60 | - | 40 | - | - | - | - | - | - | - |
| 8 | 30 | 30 | 40 | - | - | - | - | - | - | - |
| 9 | 50 | - | 50 - | - | - | - | - | - | - | - |
| 10 | 30 | 20 | 30 | 20 | - | - | - | - | - | - |
| 11 | 40 | - | 60 | - | - | - | - | - | - | - |
| 12 | 40 | - | 30 | 30 | - | - | - | - | - | - |
| 13 | 40 | - | 20 | - | 30 | - | - | - | - | 5 |
| 14 | 20 | 20 | 40 | - | - | - | 20 | - | - | - |
| 15 | - | 40 | 60 | - | - | - | - | - | - | - |
| 16 | - | 40 | 55 | - | - | - | - | - | - | 5 |
| 17 | - | 40 | 50 | 10 | - | - | - | - | - | - |
| 18 | - | 40 | 30 | 30 | - | - | - | - | - | - |
| 19 | - | 40 | 30 | - | 20 | - | - | - | - | 10 |
| 20 | - | 35 | 35 | - | 15 | - | 5 | - | - | 5 |
| 21 | 30 | - | 70 | - | - | - | - | - | - | - |
| 22 | 30 | - | 65 | - | - | - | 5 | - | - | - |
| 23 | 30 | - | 60 | - | - | - | 10 | - | - | - |
| 24 | 30 | - | 40 | 30 | - | - | - | - | - | - |
| 25 | - | 30 | 70 | - | - | - | - | - | - | - |
| 26 | - | 30 | 30 | - | 30 | - | - | - | - | 10 |
| 27 | 25 | - | 55 | 20 | - | - | - | - | - | - |
| 28 | - | 25 | 40 | - | 30 | - | - | - | - | 5 |
| 29 | 20 | - | 60 | 20 | - | - | - | - | - | - |
| 30 | 20 | - | 40 | 20 | - | - | 20 | - | - | - |
| 31 | - | 20 | 40 | - | 35 | - | - | - | - | 5 |
| 32 | - | 20 | - | 30 | 35 | - | 5 | - | - | 10 |
| 33 | 95 | - | - | - | - | - | 5 | - | - | - |
| 34 | 50 | 15 | - | - | 20 | - | - | 5 | - | 10 |
| 35 | 30 | 30 | - | - | 20 | - | - | 10 | - | 10 |
| 36 | 40 | - | 58 | - | - | - | - | 2 | - | - |
| 37 | - | 30 | 35 | - | 20 | - | - | 5 | - | 10 |
| 38 | - | 30 | - | - | 50 | - | 5 | 5 | - | 10 |
| 39 | 80 | - | - | - | - | - | - | - | 20 | - |
| 40 | 30 | 30 | - | - | 20 | - | - | - | 20 | - |
| 41 | 50 | - | 30 | - | - | - | - | - | 20 | - |
| 42 | 40 | - | 25 | - | 25 | - | - | - | 10 | - |
| 43 | - | 30 | 60 | - | - | - | - | - | 10 | - |
| 44 | - | 30 | 30 | - | 30 | - | - | - | 10 | - |
| 45 | - | 30 | 30 | - | 30 | - | 5 | - | 5 | - |
| 46 | - | 30 | 30 | - | 30 | - | - | 3 | 7 | - |
| 47 | - | 25 | 70 | - | - | - | - | - | 5 | - |
| 48 | - | 25 | 35 | - | 30 | - | - | 2 | 8 | - |
| 49 | - | 20 | 70 | - | - | - | - | - | 10 | - |
| 50 | - | 20 | 40 | - | 30 | - | - | - | 10 | - |

| | | | | | | | | | | |
|---|---|---|---|---|---|---|---|---|---|---|
| AM Acrylamid MAM Methacrylamid VP N-Vinylpyrrolidon VCap N-Vinylcaprolactam VFA N-Vinylformamid DMAA Dimethylacrylamid 350-MA Polyethylenglycolmethacrylat (Mn=350) Q-DMAEMA Dimethylaminoethylmethacrylat-Dimethylsulfat C-Dry MD1934 abgebaute Stärke (Dextrose Äquivalente DE = 38; Fa. Cerestar) PVOH teilverseifter Polyvinylalkohol (Mowiol®4-88, Fa. Clariant) | | | | | | | | | | |

Allgemeine Herstellungsvorschrift für Beispiele 51 bis 65: Lösungspolymerisation mit Nachpolymerisation und Wasserdampf-Behandlung (Beispiel 63)

| | | |
|---|---|---|
| Zulauf 1: | Monomerengemisch aus: | |
| | 120 g | Vinylpyrrolidon und |
| | 2 g | Vinylimidazol |
| | | |
| Zulauf 2: | 466,7 g | einer 15%igen wässrigen Lösung von Methacrylamid (= 70 g Methacrylamid) |
| | | |
| Zulauf 3: | Initiatorlösung aus: | |
| | 2 g | Wako V 50 [2,2'-Azobis(2-amidinopropan)-dihydrochlorid] und |
| | 18 g | Wasser |
| | | |
| Zulauf 4: | Initiatorlösung aus: | |
| | 2 g | Wako V 50 [2,2'-Azobis(2-amidinopropan)-dihydrochlorid] und |
| | 18 g | Wasser |
| | | |
| Zulauf 5: | 3 g | 25%ige Milchsäure-Lösung |
| | | |
| Vorlage: | 26,7 g | einer 50%igen wässrigen Polyvinylalkohol-LÖsung (= 8 g Mowiol® 4-88) |

In einer Rührapparatur mit Rückflusskühler, Innenthermometer und 4 separaten zulaufvorrichtungen wurden 30,5 g von Zulauf 1, 117 g von Zulauf 2 und 4 g von Zulauf 3 in 150 g Wasser vorgelegt und die Mischung unter Rühren auf ca. 65 °C aufgeheizt. Nach dem Anpolymerisieren, erkennbar an einer beginnenden Viskositätserhöhung, wurde bei 65 °C der Rest von Zulauf 1 innerhalb von 3 Stunden, der Rest von Zulauf 2 in 5 Stunden und der Rest von Zulauf 3 innerhalb von 6 Stunden zugegeben, wobei die Innentemperatur auf ca. 67 °C erhöht wurde. Nach dem Ende der Zugabe wurde das Reaktionsgemisch noch ca. 2 Stunden bei dieser Temperatur belassen. Die Polymerlösung wurde mit Wasserdampf 30 Minuten behandelt. Anschließend wurde der Zulauf 4 innerhalb von 30 Minuten und Zulauf 5 innerhalb von 5 Minuten hinzugefügt, und die Polymerlösung noch ca. drei Stunden bei einer Temperatur von ca. 70 °C nachpolymerisiert. Man erhielt ca. 800 g einer ca. 22%igen Polymerlösung. Die Lösung wurde mit Wasserdampf etwa 1 Stunde behandelt und bei einer Temperatur von ca. 100 °C noch 2 Stunden gerührt.

Zum Stabilisieren wurde die Lösung mit 100 ppm Euxyl K100 der Fa. Schülke & Mayr (5-Chlor-2-methyl-3-(2)-isothiazolon / 2-Methyl-3-(2H)-isothiazolon / Benzylalkohol) versetzt.

Pulverförmige Produkte wurden durch Sprühtrocknen oder Gefriertrocknen erhalten.

Analog hierzu wurden alle Produkte, die in der folgenden Tabelle 2 aufgeführt sind, polymerisiert.

**Tabelle 2:**

| Bsp.Nr. | MAM | VP | VCap | DMAA | 350-MA | VI | PVOH |
|---|---|---|---|---|---|---|---|
| 51 | 5 | 70 | 25 | -- | -- | -- | -- |
| 52 | 10 | 70 | 10 | -- | 10 | -- | -- |
| 53 | 10 | 70 | 15 | -- | -- | 5 | -- |
| 54 | 10 | 67 | 20 | -- | -- | 3 | -- |
| 55 | 20 | 70 | -- | 10 | -- | -- | -- |
| 56 | 20 | 70 | -- | -- | 5 | 5 | -- |
| 57 | 20 | 68 | 10 | -- | -- | 2 | -- |
| 58 | 20 | 70 | -- | -- | -- | 4 | 6 |
| 59 | 20 | 72 | -- | -- | -- | 3 | 5 |
| 60 | 25 | 62 | 10 | -- | -- | 3 | -- |
| 61 | 25 | 72 | -- | -- | -- | 3 | -- |
| 62 | 30 | 68 | -- | -- | -- | 2 | -- |
| 63 | 35 | 60 | -- | -- | -- | 1 | 4 |
| 64 | 38 | 60 | -- | -- | -- | 2 | -- |
| 65 | 40 | 58 | -- | -- | -- | 2 | -- |

| | | | | | | | |
|---|---|---|---|---|---|---|---|
| VI: Vinylimidazol VCap: N-Vinylcaprolactam DMAA: Dimethylacrylamid 350-MA: Polyethylenglycolmethacrylat (Mₙ350) PVOH: teilverseifter Polyvinylalkohol (Mowiol®4-88, Fa. Clariant) | | | | | | | |

### Anwendungstechnische Eigenschaften

### Standardformulierung:

Aus 0,5 Gew.-% eines handelsüblichen Polyacrylsäureverdickers (Carbopol 940, Fa. BFGoodrich), neutralisiert mit Triethanolamin (TEA) wird ein Gel formuliert, das bei Anwendung auf dem Haar im Wesentlichen keine Conditionier- oder Festigungswirkung zeigt. Die anwendungstechnischen Eigenschaften sind in Tabelle 3, Vergleichsbeispiel A, wiedergegeben.

### Vergleichsbeispiele B-D:

Der Gelformulierung aus der Standardformulierung werden je 3 Gew.-% eines handelsüblichen Haarpolymers (Bsp. B: Polyacrylsäureamid, C: Polyvinylformamid, D: Polyvinylpyrrolidon) zugegeben. Die anwendungstechnischen Eigenschaften sind in Tabelle 2 wiedergegeben. Die Produkte sind hinsichtlich ihrer Klebrigkeit noch verbesserungswürdig.

### Erfindungsgemäß:

Der Gelformulierung aus der Standardformulierung werden je 3 Gew.-% der Copolymere 1 bis 67 als haarkosmetischer Wirkstoff zugegeben. Es resultieren klare Formulierungen mit guter Conditionier- bzw. Festigerwirkung. Die anwendungstechnischen Eigenschaften sind ebenfalls in Tabelle 3 wiedergegeben.

### Bewertung:

### A) Klarheit

| Note | Klarheit | |
|---|---|---|
| 1 | klar | (Referenz: Carbopol 940; Polyvinylformamid mit K-Wert=40) |
| 1-2 | fast klar | (Referenz: Luviskol®VA 64) |
| 2 | handklar | (klar beim Ausbilden eines dünnen Films auf der Hand; Referenz: Polyvinylformamid mit K-Wert=110; Luviskol®VA 73) |
| 3 | leicht trüb | (Referenz: Polyvinylpyrrolidon K 90) |
| 4 | trüb | (Referenz: Polyvinylalkohol, z.B. Mowiol®4-88) |
| 5 | milchig | |

### B) Viskosität

| Note | Viskosität |
|---|---|
| 1 | sehr fest (Referenz: Gel aus 0,5 % Carbopol 940/TEA) |
| 2 | fest |
| 3 | mäßig fest |
| 4 | fließend |
| 5 | niedrigviskos |

### C) Klebrigkeit

Die Klebrigkeit wurde bei einer relativen Luftfeuchtigkeit von 75 % und bei Umgebungstemperatur direkt an getrockneten Filmen der Gelformulierungen bestimmt.

| Note | Klebrigkeit |
|---|---|
| 1 | nicht klebrig |
| 2 | leicht klebrig |
| 3 | mäßig klebrig |
| 4 | klebrig |
| 5 | sehr klebrig |

**Tabelle 3:**

| Bsp.-Nr. | A-Note (Klarheit des Gels) | B-Note (Viskosität des Gels) | C-Note (Klebrigkeit des getrockneten Films des Gels) |
|---|---|---|---|
| A | Gel aus 0,5 % Carbopol 940/TEA | | |
| | 1 | 1-2 | 1 (harter, brüchiger, unvollständiger Film) |
| B | Gel aus 3 % Polymer + 0,5 % Carbopol 940/TEA | | |
| | 3-4 | 12 | 1-2 |
| C | Gel aus 3 % Polymer + 0,5 % Carbopol 940/TEA | | |
| | 1 | 1-2 | 2-3 |
| D | Gel aus 3 % Polymer + 0,5 % Carbopol 940/TEA | | |
| | 1 | 1-2 | 3 |
| 1 | 2 | 1-2 | 2 |
| 2 | 1-2 | 1 | 1-2 |
| 3 | 1-2 | 1-2 | 2 |
| 4 | 1-2 | 1-2 | 2 |
| 5 | 1-2 | 1-2 | 1-2 |
| 6 | 1-2 | 1-2 | 2 |
| 7 | 1-2 | 1-2 | 1-2 |
| 8 | 1-2 | 1-2 | 1-2 |
| 9 | 1 | 1-2 | 1-2 |
| 10 | 2 | 1-2 | 1-2 |
| 11 | 1 | 1-2 | 1-2 |
| 12 | 2 | 1 | 1 |
| 13 | 1 | 1 | 1-2 |
| 14 | 2 | 2 | 2 |
| 15 | 1 | 1-2 | 1-2 |
| 16 | 1 | 1 | 1 |
| 17 | 1 | 1 | 1 |
| 18 | 2 | 1 | 1-2 |
| 19 | 1-2 | 1 | 1-2 |
| 20 | 1-2 | 1 | 1-2 |
| 21 | 1 | 1-2 | 2 |
| 22 | 1 | 1-2 | 2 |
| 23 | 1-2 | 1-2 | 2 |
| 24 | 1-2 | 1 | 1-2 |
| 25 | 1-2 | 1-2 | 1-2 |
| 26 | 1 | 1-2 | 1-2 |
| 27 | 1-2 | 1-2 | 1-2 |
| 28 | 1 | 1-2 | 1-2 |
| 29 | 1-2 | 1-2 | 1-2 |
| 30 | 1-2 | 1-2 | 2 |
| 31 | 1 | 1 | 1-2 |
| 32 | 1-2 | 1-2 | 1-2 |
| 33 | 1-2 | 1-2 | 2 |
| 34 | 1-2 | 2 | 1-2 |
| 35 | 1-2 | 2 | 2 |
| 36 | 1-2 | 1-2 | 1-2 |
| 37 | 1-2 | 1-2 | 1-2 |
| 38 | 1-2 | 1-2 | 2 |
| 39 | 1-2 | 1 | 2 |
| 40 | 1 | 1 | 2 |
| 41 | 1-2 | 1 | 2 |
| 42 | 1 | 1-2 | 1-2 |
| 43 | 1-2 | 1-2 | 1-2 |
| 44 | 1-2 | 1 | 1 |
| 45 | 1 | 1 | 1-2 |
| 46 | 1 | 1 | 1-2 |
| 47 | 1-2 | 1-2 | 1-2 |
| 48 | 1 | 1-2 | 1-2 |
| 49 | 1 | 1-2 | 1-2 |
| 50 | 1 | 1 | 1-2 |
| 51 | 1-2 | 1 | 1 |
| 52 | 1 | 1 | 1-2 |
| 53 | 1 | 1 | 1-2 |
| 54 | 1 | 1 | 1 |
| 55 | 1 | 1 | 1-2 |
| 56 | 1 | 1 | 1-2 |
| 57 | 1 | 1 | 1 |
| 58 | 1 | 1 | 1 |
| 59 | 1 | 1 | 1 |
| 60 | 1-2 | 1 | 1 |
| 61 | 1 | 1 | 1 |
| 62 | 1 | 1 | 1 |
| 63 | 1 | 1 | 1 |
| 64 | 1 | 1 | 1 |
| 65 | 1 | 1 | 1 |

Die Klarkeit der Gele kann durch Zugabe von bis zu 20 Gew.-% Ethanol noch verbessert werden.

### Verwendung in der Haarkosmetik:

### 1) Haargele mit einem anionischen Verdicker: Beispiele Nr. 1-50

| Phase 1: | [%] | CTFA |
|---|---|---|
| Polymer 1-50 (30 %ige wässrige Lösung) | 10,0 | |
| Glycerin | 0,3 | |
| Wasser dest. | 39,2 | |
| Weitere Zusatzstoffe: Konservierungs-mittel, lösliches ethoxyliertes Silikon, Parfüm | | q.s. |
| | | |

| Phase 2: | | |
|---|---|---|
| Carbopol 940 (1 %ige wässrige Suspension) | 30,0 | Carbomer |
| Triethanolamin | 0,5 | |
| Wasser dest. | 20,0 | |

Zur Herstellung des Haargels werden die Komponenten eingewogen und homogenisiert. Dabei bildet die Phase 2 ein klares, festes Gel, in das Phase 1 langsam eingerührt wird.

### 2) Haargele mit einem weiteren Festigerpolymer und anionischem Verdicker: Beispiele Nr. 51-100

| Phase 1: | [%] | CTFA |
|---|---|---|
| Polymer 1-50 (30 %ige wässrige Lösung) | 7,0 | |
| Luviskol VA 64 | 1,0 | Vinylpyrrolidonvinylacetat-Copolymer |
| Univul MS 40 | 0,2 | Benzophenon-4 |
| Glycerin | 0,2 | |
| D-Panthenol USP | 0,1 | Panthenol |
| Ethanol | 20,0 | |
| Wasser dest. | 21,0 | |
| Weitere Zusatzstoffe: Konservierungsmittel, lösliches ethoxyliertes Silikon, Parfüm | | q.s. |
| | | |

| Phase 2: | | |
|---|---|---|
| Carbopol 940 (1 %ige wässrige Suspension) | 30,0 | Carbomer |
| Triethanolamin | 0,5 | |
| Wasser dest. | 20,0 | |

Herstellung: Einwiegen und Homogenisieren. Phase 2 bildet ein klares, festes Gel. Phase 1 langsam in Phase 2 einrühren.

### 3) Flüssige Haargele: Beispiele Nr. 101-138

| | [%] | CTFA |
|---|---|---|
| Polymer 1-38 (30 %ige wässrige Lösung) | 5,0 | |
| Glycerin | 0,3 | |
| Natrosol 250 L (2 %ige wässrige. Lösung) | 25,0 | Hydroxyethylcellulose (Fa. Hercules) |
| C-Dry MD 1915 (10 %ige wässr. Lösung) | 25,0 | abgebaute Stärke (Fa. Cerestar) |
| Wasser dest. | 44,7 | |
| Weitere Zusatzstoffe: Konservierungsmittel, lösliches ethoxyliertes Silikon, Parfüm | q.s. | |

Herstellung: Einwiegen und bei Raumtemperatur langsam Homogenisieren.

### 4) Wässrige Handpumpen-Sprays: Beispiele Nr. 139-175

| | [%] | CTFA |
|---|---|---|
| Polymer 14-50 (30 %ige wässrige Lösung) | 10,0 | |
| Luviset®PUR (30%ige Wasser/Ethanol-Lsg.) | 5,0 | (PU-Dispersion Fa. BASF) |
| C-Dry MD 1915 (10 %ige wässr. Lösung) | 5,0 | abgebaute Stärke (Fa. Cerestar) |
| Wasser dest. | 45,0 | |
| Weitere Zusatzstoffe: Konservierungs-mittel, lösliches ethoxyliertes Silikon, Parfüm | | q.s. |

Herstellung: Einwiegen und bei Raumtemperatur langsam Homogenisieren.

### 5) VOC 55 Handpumpen-Spray: Beispiele Nr. 176-194

| | [%] | CTFA |
|---|---|---|
| Polymer 4-10, 11, 12, 15-18, 21-25, 27 (30 %ige wässrige Lösung) | 10,0 | |
| Wasser dest. | 35,0 | |
| Ethanol | 55,0 | |
| Weitere Zusatzstoffe: Konservierungsmittel, lösliches ethoxyliertes Silikon, Parfüm | | q.s. |

### 6) VOC 55 Aerosol-Haarspray: Beispiele Nr. 195-213

| | [%] | CTFA |
|---|---|---|
| Polymer 4-10, 11, 12, 15-18, 21-25, 27 (30 %ige wässrige Lösung) | 5,0 | |
| Luviset®PUR (30%ige Wasser/Ethanol-Lsg.) | 5,0 | (PU-Dispersion Fa. BASF) |
| Wasser dest. | 35,5 | |
| Dimethylether | 30,0 | |
| Ethanol | 24,5 | |
| Weitere Zusatzstoffe: Konservierungsmittel, lösliches ethoxyliertes Silikon, Parfüm | | q.s. |

### 7) Schaumfestiger: Beispiele Nr. 214-232

| | [%] | CTFA |
|---|---|---|
| Polymer 4-10, 11, 12, 15-18, 21-25, 27 (30 %ige wässrige Lösung) | 5,0 | |
| Cremophor A 25 (Ceteareth 25/BASF) | 0,2 | |
| Comperlan KD (Coamide DEA/Henkel) | 0,1 | |
| Wasser dest. | 74,7 | |
| Dimethylether | 10,0 | |
| Weitere Zusatzstoffe: Konservierungsmittel, lösliches ethoxyliertes Silikon, Parfüm | | q.s. |

Herstellung: Einwiegen und unter Rühren lösen. Abfüllen und Treibgas zusetzen.

### 8) Shampoo: Beispiele Nr. 233-272

### Conditioner Shampoo:

| | [%] | CTFA |
|---|---|---|
| A) Texapon NSO 28 %ig (Natriumlaurylsulfat/Henkel) | 50,0 | |
| Comperlan KD (Coamide DEA/Henkel) | 1,0 | |
| Polymer 1-38, 46, 48 (30 %ige wässrige Lösung) | 3,0 | |
| Wasser dest. | 17,0 | |
| q.s. Parfümöl | | |
| | | |
| B) Wasser | 27,5 | |
| Natriumchlorid | 1,5 | |
| q.s. Konservierungsmittel | | |

Herstellung: Einwiegen und unter Rühren Phasen A) und B) getrtennt lösen und mischen. Phase B) langsam in Phase A) einrühren.

### Verwendung in der Hautkosmetik:

### 9) Standard O/W-Creme: Beispiele Nr. 273-290

| Ölphase: | [%] | CTFA |
|---|---|---|
| Cremophor A6 | 3,5 | Ceteareth-6 und Stearylalkohol |
| Cremophor A25 | 3,5 | Ceteareth-25 |
| Glycerinmonostearat s.e. | 2,5 Stearate | Glyceryl |
| Paraffinöl | 7,5 | Paraffin Oil |
| Cetylalkohol | 2,5 | Cetyl Alkohol |
| Luvitol EHO | 3,2 | Cetearyloctanoat |
| Vitamin-E-acetate | 1,0 | Tocopheryl Acetate |
| Nip-Nip | 0,1 | Methyl- und Propyl-4-hydroxybenzoate (7:3) |
| | | |

| Wasserphase: | [%] | |
|---|---|---|
| Polymer Nr. 3, 14, 16, 20-23, 30-38, 45, 46 (30 %ige wässrige Lösung) | 3,0 | |
| Wasser | 74,6 | |
| 1,2-Propylenglycol | 1,5 | |
| Germall II | 0,1 | Imidazolidinyl-Urea |

Herstellung: Einwiegen und unter Rühren die Ölphase und die Wasserphase getrennt bei einer Temperatur von 80°C homogenisieren. Die Wasserphase langsam in die Ölphase einrühren. Unter Rühren langsam auf Raumtemperatur abkühlen.

### 10) Tageslotion: Beispiele Nr. 291-308

| Ölphase: | [%] | CTFA |
|---|---|---|
| Cremophor A6 | 1,5 | Ceteareth-6 und Stearylalkohol |
| Cremophor A25 | 1,5 | Ceteareth-25 |
| Glycerinmonostearat s.e. | 5,0 Stearate | Glyceryl |
| Uvinul MS 40 | 0,5 | Benzophenone-4 |
| Paraffinöl | 3,5 | Paraffin Oil |
| Cetylalkohol | 0,5 | Cetyl Alkohol |
| Luvitol EHO | 10,0 Octanoat | Cetearyl |
| D-Panthenol 50P | 3,0 | Panthenol und Propylenglycol |
| Vitamin-E-acetate | 1,0 | Tocopheryl Acetate |
| Tegiloxan 100 | 0,3 | Dimethicon |
| Nip-Nip | 0,1 | Methyl- und Propyl-4-hydroxybenzoate (7:3) |
| | | |

| Wasserphase: | [%] | |
|---|---|---|
| Polymer Nr. 4, 12, 17, 18, 20, 24-28, 33-34, 38-48, 55-56, (30 %ige wässrige Lösung) | 1,5 | |
| Wasser | 70,0 | |
| 1,2-Propylenglycol | 1,5 | |
| Germall II | 0,1 | Imidazolidinyl-Harnstoff |

Herstellung: Einwiegen und unter Rühren die Ölphase und die Wasserphase getrennt bei einer Temperatur von 80°C homogenisieren. Die Wasserphase langsam in die Ölphase einrühren. Unter Rühren langsam auf Raumtemperatur abkühlen.

## Patentansprüche

1. Kosmetisches oder pharmazeutisches Mittel, enthaltend
A) wenigstens ein wasserlösliches oder wasserdispergierbares Copolymer, das erhältlich ist durch radikalische Copolymerisation von
a) 5 bis 90 Gew.-%, bezogen auf das Gesamtgewicht der Komponenten a) bis d), Acrylsäureamid und/oder Methacrylsäureamid,
b) 5 bis 85 Gew.-%, bezogen auf das Gesamtgewicht der Komponenten a) bis d), wenigstens einer α,β-ethylenisch ungesättigten amidgruppenhaltigen Verbindung der allgemeinen Formel I 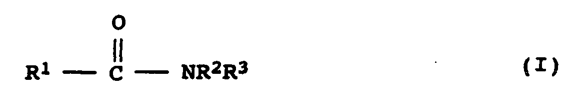 wobei
R¹ für eine Gruppe der Formel CH₂=CR⁴- mit R⁴ = H oder C₁-C₄-Alkyl steht und R² und R³ unabhängig voneinander für H, Alkyl, Cycloalkyl, Heterocycloalkyl, Aryl oder Hetaryl stehen, mit der Maßgabe, das einer der Reste R² und R³ von H verschieden ist, oder R² und R3 gemeinsam mit dem Stickstoffatom, an das sie gebunden sind, für einen fünf- bis achtgliedrigen Heterocyclus stehen,
oder R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind für ein Lactam mit 5 bis 8 Ringatomen stehen,
c) 0 bis 40 Gew.-%, bezogen auf das Gesamtgewicht der Komponenten a) bis d), wenigstens einer von den Komponenten a) und b) verschiedenen, damit copolymerisierbaren ungesättigten wasserlöslichen Verbindung,
die ausgewählt ist unter Estern α,β-ethylenisch ungesättigter Mono- und Dicarbonsäuren mit Aminoalkoholen, deren N-Alkyl- und N,N-Dialkylderivaten; Estern von Vinylalkohol mit Monocarbonsäuren; vinyl- und allylsubstituierten heteroaromatischen Verbindungen; Amiden α,β-ethylenisch ungesättigter Mono- und Dicarbonsäuren mit Diaminen, die eine tertiäre und eine primäre oder sekundäre Aminogruppe aufweisen; Polyetheracrylaten und Mischungen davon,
wobei der Gewichtsmengenanteil der Summe der Komponenten b) und c) wenigstens 5 Gew.-% beträgt,
gegebenenfalls in Gegenwart von bis zu 25 Gew.-%, bezogen auf das Gesamtgewicht der Komponenten a) bis d), wenigstens einer wasserlöslichen Komponente d), die ausgewählt ist unter
d1) polyetherhaltigen Verbindungen,
d2) Polymeren, die mindestens 50 Gew.-% Wiederholungseinheiten aufweisen, die sich von Vinylalkohol ableiten,
d3) Stärke und Stärkederivaten,
und Mischungen davon, und
B) wenigstens einen kosmetisch akzeptablen Träger.

2. Mittel nach Anspruch 1, wobei die Komponente b) wenigstens eine Verbindung umfaßt, die ausgewählt ist unter N-Vinyllactamen, N-vinylamiden gesättigter Monocarbonsäuren, N-Alkyl- und N,N-Dialkylamiden α,β-ethylenisch ungesättigter Monocarbonsäuren und Mischungen davon.

3. Mittel nach einem der vorhergehenden Ansprüche, wobei das Copolymer A) keine säuregruppenhaltigen Monomere einpolymerisiert enthält.

4. Mittel nach einem der vorhergehenden Ansprüche, wobei die Komponente B) ausgewählt ist unter
i) Wasser,
ii) wassermischbaren organischen Lösungsmitteln, vorzugsweise C₁-C₄-Alkanolen,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen
und Mischungen davon.

5. Mittel nach einem der vorhergehenden Ansprüche, enthaltend außerdem wenigstens einen von Copolymer A verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

6. Mittel nach einem der vorhergehenden Ansprüche in Form eines Gels, Schaums, Sprays, einer Salbe, Creme, Emulsion, Suspension, Lotion, Milch oder Paste.

7. Copolymer A) wie in einem der Ansprüche 1 bis 3 definiert, erhältlich durch radikalische Polymerisation von
a) 20 bis 40 Gew.-% Methacrylsäureamid,
b) 40 bis 70 Gew.-% Vinylpyrrolidon,
in Gegenwart von 1 bis 20 Gew.-% Polymeren d2) und/oder Stärke und Stärkederivaten d3).

8. Copolymer A) wie in einem der Ansprüche 1 bis 3 definiert, erhältlich durch radikalische polymerisation von
a) 30 bis 40 Gew.-% Methacrylsäureamid,
b) 20 bis 60 Gew.-% Vinylpyrrolidon und
1 bis 20 Gew.-% Vinylcaprolactam.

9. Copolymer A) wie in einem der Ansprüche 1 bis 3 definiert, erhältlich durch radikalische copolymerisation von
a) 5 bis 50 Gew.-%, bezogen auf das Gesamtgewicht der Komponenten a) bis d), Methacrylsäureamid,
b) 40 bis 85 Gew.-%, bezogen auf das Gesamtgewicht der Komponenten a) bis d), wenigstens einer Verbindung, ausgewählt unter Vinylpyrrolidon, Vinylcaprolactam, N,N-Dimethylacrylamid und Mischungen davon,
c) 0,2 bis 20 Gew.-%, bezogen auf das Gesamtgewicht der Komponenten a) bis d) wenigstens einer von a) und b) verschiedenen, damit copolymerisierbaren ungesättigten wasserlöslichen Verbindung, die ausgewählt ist unter Vinylimidazol und Derivaten davon, Polyetheracrylaten und Mischungen davon,
gegebenenfalls in Gegenwart von bis zu 10 Gew.-%, bezogen auf das Gesamtgewicht der Komponenten a) bis d), Polymeren d2), die sich von Vinylalkohol ableiten, und gegebenenfalls in Gegenwart von bis zu 1 Gew.-%, bezogen auf das Gesamtgewicht der Komponenten a) bis d), wenigstens eines Vernetzers.

10. Copolymer A) wie in einem der Ansprüche 1 bis 3 definiert, erhältlich durch radikalische Polymerisation von
a) 7 bis 45 Gew.-% Methacrylsäureamid,
b) 50 bis 80 Gew.-% wenigstens einer Verbindung ausgewählt unter Vinylpyrrolidon, Vinylcaprolactam, N,N-Dimethylacrylamid und Mischungen davon,
c) 0,3 bis 10 Gew.-% wenigstens einer Verbindung, die ausgewählt ist unter Vinylimidazol und Derivaten davon, Polyetheracrylaten und Mischungen davon,
in Gegenwart von 0,1 bis 10 Gew.-% Polymeren d2), die sich von Vinylalkohol ableiten.

11. Copolymer A) wie in einem der Ansprüche 1 bis 3 definiert, erhältlich durch radikalische polymerisation von
a) 10 bis 45 Gew.-% Methacrylsäureamid,
b) 50 bis 80 Gew.-% Vinylpyrrolidon und Vinylcaprolactam und
c) 0,3 bis 10 Gew.-% Vinylimidazol und/oder eines Derivats davon.

12. Copolymer A) nach Anspruch 9, erhältlich durch radikalische Polymerisation von
a) 10 bis 45 Gew.-% Methacrylsäureamid,
b) 50 bis 80 Gew.-% Vinylpyrrolidon und
c) 0,5 bis 5 Gew.-% Vinylimidazol.

13. Verfahren zur Herstellung eines Copolymeren A, wie in einem der Ansprüche 1 bis 3 und 7 bis 12, definiert, durch radikalische Polymerisation der Monomere a) mit wenigstens einem weiteren Monomer ausgewählt unter den Monomeren b) und c) gegebenenfalls in Gegenwart von bis zu 25 Gew.-%, bezogen auf das Gesamtgewicht der Komponenten a) bis d), einer wasserlöslichen Komponente d), **dadurch gekennzeichnet, dass** man die Polymerisation in einem wässrigen Lösungsmittel durchführt.

14. Verfahren nach Anspruch 13, **dadurch gekennzeichnet, dass** man die Polymerisation bei einem pH-Wert im Bereich von 6 bis 8 durchführt.

15. Verfahren nach Anspruch 14, **dadurch gekennzeichnet, dass** man die Polymerisation bei einem pH-Wert im Berich von 6,4 bis 7,4 durchführt.

16. Verwendung eines Copolymers A), wie in einem der Ansprüche 1 bis 3 und 7 bis 12 definiert, in Hautreinigungsmitteln, Mitteln zur Pflege und zum Schutz der Haut, Nagelpflegemitteln, Zubereitungen für die dekorative Kosmetik und Haarbehandlungsmitteln.

17. Verwendung nach Anspruch 16 in Haarbehandlungsmitteln als Festiger und/oder als Conditioner.

18. Verwendung nach Anspruch 17, wobei das Mittel in Form eines Haargels, Shampoos, Schaumfestigers, Haarwassers, Haarsprays oder Haarschaums vorliegt.

19. Verwendung eines Copolymers A), wie in einem der Ansprüche 1 bis 3 und 7 bis 12 definiert, als Hilfsmittel in der Pharmazie, bevorzugt als oder in Beschichtungsmittel(n) für feste Arzneiformen sowie als oder in Beschichtungsmittel(n) für die Textil-, Papier-, Druck- und Lederindustrie.

20. Verwendung eines wasserlöslichen Copolymers A), wie in einem der Ansprüche 1 bis 3 und 7 bis 12 definiert, als Pfropfgrundlage, Polymeremulgator oder Schutzkolloid.

## Claims

1. A cosmetic or pharmaceutical composition comprising
A) at least one water-soluble or water-dispersible copolymer obtainable by free-radical copolymerization of
a) 5 to 90% by weight, based on the total weight of components a) to d), of acrylamide and/or methacrylamide,
b) 5 to 85% by weight, based on the total weight of components a) to d), of at least one α,β-ethylenically unsaturated amide-containing compound of the formula I 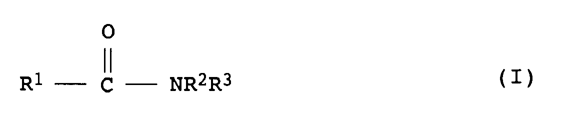 where
R¹ is a group of the formula CH₂=CR⁴- where R⁴ = H or C₁-C₄-alkyl, and R² and R³, independently of one another, are each H, alkyl, cycloalkyl, heterocycloalkyl, aryl or hetaryl, with the proviso that one of the radicals R² and R³ is different from H, or R² and R³ together with the nitrogen atom to which they are bonded are a five- to eight-membered heterocycle,
or R² is a group of the formula CH₂=CR⁴- and R¹ and R³, independently of one another, are each H, alkyl, cycloalkyl, heterocycloalkyl, aryl or hetaryl, or R¹ and R³ together with the amide group to which they are bonded are a lactam with 5 to 8 ring atoms,
c) 0 to 40% by weight, based on the total weight of components a) to d), of at least one unsaturated water-soluble compound which is different from components a) and b) and copolymerizable therewith,chosen from esters of α,β-ethylenically unsaturated mono- and dicarboxylic acids with aminoalcohols, their N-alkyl- and N,N-dialkyl derivatives; esters of vinyl alcohol with monocarboxylic acids; vinyl- and allyl-substituted heteroaromatic compounds; amides of α,β-ethylenically unsaturated mono- and dicarboxylic acids with diamines which have a tertiary and a primary or secondary amino group; polyether acrylates and mixtures thereof,
where the proportion by weight of the sum of components b) and c) is at least 5% by weight,
if appropriate in the presence of up to 25% by weight, based on the total weight of components a) to d), of at least one water-soluble component d), which is chosen from
d1) polyether-containing compounds,
d2) polymers which have at least 50% by weight repeat units derived from vinyl alcohol,
d3) starch and starch derivatives,
and mixtures thereof, and
B) at least one cosmetically acceptable carrier.

2. The composition according to claim 1, where component b) comprises at least one compound chosen from N-vinyllactams, N-vinylamides of saturated monocarboxylic acids, N-alkyl- and N,N-dialkylamides of α,β-ethylenically unsaturated monocarboxylic acids and mixtures thereof.

3. The composition according to either of the preceding claims, where copolymer A) does not comprise any copolymerized acid-containing monomers.

4. The composition according to any of the preceding claims, where component B) is chosen from
i) water,
ii) water-miscible organic solvents, preferably C₁-C₄-alkanols,
iii)oils, fats, waxes,
iv) esters of C₆-C₃₀-monocarboxylic acids with mono-, di- or trihydric alcohols different from iii),
v) saturated acyclic and cyclic hydrocarbons,
vi) fatty acids,
vii)fatty alcohols
and mixtures thereof.

5. The composition according to any of the preceding claims, further comprising at least one constituent different from copolymer A which is chosen from cosmetically active ingredients, emulsufiers, surfactants, preservatives, perfume oils, thickeners, hair polymers, hair and skin conditioners, graft polymers, water-soluble or dispersible silicone-containing polymers, light protection agents, bleaching agents, gel formers, care agents, colorants, tinting agents, tanning agents, dyes, pigments, consistency-imparting agents, humectants, refatting agents, collagen, protein hydrolysates, lipids, antioxidants, antifoams, antistats, emollients and softeners.

6. The composition according to any of the preceding claims in the form of a gel, foam, spray, an ointment, cream, emulsion, suspension, lotion, milk or paste.

7. The copolymer A) as defined in any of claims 1 to 3, obtainable by free-radical polymerization of
a) 20 to 40% by weight of methacrylamide,
b) 40 to 70% by weight of vinylpyrrolidone,
in the presence of from 1 to 20% by weight of polymers d2) and/or starch and starch derivatives d3).

8. The copolymer A) as defined in any of claims 1 to 3, obtainable by free-radical polymerization of
a) 30 to 40% by weight of methacrylamide,
b) 20 to 60% by weight of vinylpyrrolidone and
1 to 20% by weight of vinylcaprolactam.

9. The copolymer A) as defined in any of claims 1 to 3, obtainable by free-radical copolymerization of
a) 5 to 50% by weight, based on the total weight of components a) to d), of methacrylamide,
b) 40 to 85% by weight, based on the total weight of components a) to d), of at least one compound chosen from vinylpyrrolidone, vinylcaprolactam, N,N-dimethylacrylamide and mixtures thereof,
c) 0.2 to 20% by weight, based on the total weight of components a) to d), of at least one unsaturated water-soluble compound different from a) and b) and copolymerizable therewith which is chosen from vinylimidazole and derivatives thereof, polyether acrylates and mixtures thereof,
if appropriate in the presence of up to 10% by weight, based on the total weight of components a) to d), of polymers d2) which are derived from vinyl alcohol, and if appropriate in the presence of up to 1% by weight, based on the total weight of components a) to d), of at least one crosslinker.

10. The copolymer A as defined in any of claims 1 to 3, obtainable by free-radical polymerization of
a) 7 to 45% by weight of methacrylamide,
b) 50 to 80% by weight of at least one compound chosen from vinylpyrrolidone, vinylcaprolactam, N,N-dimethylacrylamide and mixtures thereof,
c) 0.3 to 10% by weight of at least one compound chosen from vinylimidazole and derivatives thereof, polyether acrylates and mixtures thereof,
in the presence of from 0.1 to 10% by weight of polymers d2) which are derived from vinyl alcohol.

11. The copolymer A) as defined in any of claims 1 to 3, obtainable by free-radical polymerization of
a) 10 to 45% by weight of methacrylamide,
b) 50 to 80% by weight of vinylpyrrolidone and vinylcaprolactam and
c) 0.3 to 10% by weight of vinylimidazole and/or a derivative thereof.

12. The copolymer A) according to claim 9, obtainable by free-radical polymerization of
a) 10 to 45% by weight of methacrylamide,
b) 50 to 80% by weight of vinylpyrrolidone and
c) 0.5 to 5% by weight of vinylimidazole.

13. A process for the preparation of a copolymer A, as defined in any of claims 1 to 3 and 7 to 12, by free-radical polymerization of the monomers a) with at least one further monomer chosen from the monomers b) and c), if appropriate in the presence of up to 25% by weight, based on the total weight of components a) to b), of a water-soluble component d), wherein the polymerization is carried out in an aqueous solvent.

14. The process according to claim 13, wherein the polymerization is carried out at a pH in the range from 6 to 8.

15. The process according to claim 14, wherein the polymerization is carried out at a pH in the range from 6.4 to 7.4.

16. The use of a copolymer A) as defined in any of claims 1 to 3 and 7 to 12 in skin-cleansing compositions, compositions for the care and protection of the skin, nail care compositions, preparations for decorative cosmetics and hair-treatment compositions.

17. The use according to claim 16 in hair-treatment compositions as setting agents and/or as conditioners.

18. The use according to claim 17, where the composition is in the form of a hair gel, shampoo, setting foam, hair tonic, hairspray or hair foam.

19. The use of a copolymer A) as defined in any of claims 1 to 3 and 7 to 12, as an auxiliary in pharmacy, preferably as or in (a) coating(s) for solid medicaments and as or in (a) coating(s) for the textile, paper, printing and leather industry.

20. The use of a water-soluble copolymer A), as defined in any of claims 1 to 3 and 7 to 12, as graft base, polymer emulsifier or protective colloid.

## Revendications

1. Agent cosmétique ou pharmaceutique, contenant :
A) au moins un copolymère soluble dans l'eau ou dispersible dans l'eau, qui peut être obtenu par copolymérisation radicalaire de :
a) 5 à 90% en poids, par rapport au poids total des constituants a) à d), d'amide de l'acide acrylique et/ou d'amide de l'acide méthacrylique,
b) 5 à 85% en poids, par rapport au poids total des constituants a) à d), d'au moins un composé contenant des groupes amide α,β-éthyléniquement insaturés selon la formule générale I 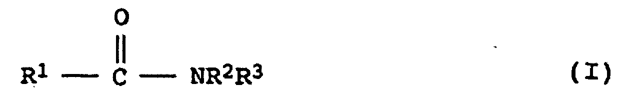 dans laquelle,
R¹ représente un groupe de formule CH₂=CR⁴- avec R⁴ = H ou un alkyle en C₁-C₄, et R² et R³ représentent indépendamment l'un de l'autre de l'hydrogène, un alkyle, un cycloalkyle, un hétérocycloalkyle, un aryle ou un hétaryle, étant entendu que l'un des radicaux R² et R³ est différent de H, ou que R² et R³ représentent ensemble avec l'atome d'azote auquel ils sont liés un hétérocycle de cinq à huit chaînons,
ou R² représente un groupe de formule CH₂=CR⁴- et R¹ et R³ représentent indépendamment l'un de l'autre de l'hydrogène, un alkyle, un cycloalkyle, un hétérocycloalkyle, un aryle ou un hétaryle, ou R¹ et R³ représentent ensemble avec le groupe amide, auquel ils sont liés, un lactame avec 5 à 8 atomes cycliques,
c) 0 à 40% en poids, par rapport au poids total des constituants a) à d), d'au moins un composé différent de l'un des constituants a) et b), et donc soluble dans l'eau, insaturé et copolymérisable, qui est sélectionné parmi des esters d'acides monocarboxyliques et dicarboxyliques α,β-éthyléniquement insaturés avec des aminoalcools, leurs dérivés N-alkyliques et N,N-dialkyliques ; des esters d'alcool vinylique avec des acides monocarboxyliques ; des composés hétéroaromatiques vinylsubstitués et allylsubstitués ; des amides d'acides monocarboxyliques et dicarboxyliques α,β-éthyléniquement insaturés avec des diamines qui présentent un groupe amino tertiaire et un groupe amino primaire ou secondaire ; des acrylates de polyéther et leurs mélanges,
la proportion quantitative en poids de la somme des constituants b) et c) s'élevant à au moins 5% en poids,
éventuellement en présence de 25% en poids maximum, par rapport au poids total des constituants a) à d), d'au moins un constituant d) soluble dans l'eau qui est sélectionné parmi :
d1) des composés comprenant des polyéthers,
d2) des polymères qui présentent au moins 50% en poids d'unités de répétition qui découlent de l'alcool vinylique,
d3) de l'amidon et des dérivés d'amidon,
et leurs mélanges, et
B) au moins un support cosmétiquement acceptable.

2. Agent suivant la revendication 1, le constituant b) comprenant au moins un composé qui est sélectionné parmi des N-vinyllactames, des N-vinylamides d'acides monocarboxyliques saturés, des N-alkylamides et des N,N-dialkylamides d'acides monocarboxyliques α,β-éthyléniquement insaturés et leurs mélanges.

3. Agent suivant l'une des revendications qui précèdent, le copolymère A) ne contenant aucun monomère contenant des groupes acides copolymèrisé.

4. Agent suivant l'une des revendications qui précèdent, le constituant B) étant sélectionné parmi :
i) l'eau,
ii) des solvants organiques miscibles dans l'eau, préférentiellement des alcanols en C₁-C₄,
iii) des huiles, des graisses, des cires,
iv) des esters différents de iii) d'acides monocarboxyliques en C₆-C₃₀ avec des alcools monovalents, bivalents ou trivalents,
v) des hydrocarbures acycliques et cycliques saturés,
vi) des acides gras,
vii) des alcools gras
et leurs mélanges.

5. Agent suivant l'une des revendications qui précède, comprenant en outre au moins un constituant différent du copolymère A, qui est sélectionné parmi des substances cosmétiquement actives, des émulsifiants, des tensioactifs, des agents de conservation, des huiles pour parfum, des épaississeurs, des polymères capillaires, des conditionneurs capillaires et cutanés, des polymères greffés, des polymères siliconés solubles dans l'eau ou dispersibles, des photostabilisants, des agents de blanchiment, des gélifiants, des produits d'entretien, des teintures, des agents nuanceurs, des agents brunissants, des colorants, des pigments, des donneurs de consistance, des produits humidifiants, des agents surgras, des collagènes, des hydrolysats protéiques, des lipides, des antioxydants, des démoussants, des antistatiques, des émollients et des plastifiants.

6. Agent suivant l'une des revendications qui précèdent sous la forme d'un gel, d'une mousse, d'un spray, d'un onguent, d'une crème, d'une émulsion, d'une suspension, d'une lotion, d'un lait ou d'une pâte.

7. Copolymère A) tel que défini dans l'une des revendications 1 à 3, qui peut être obtenu par polymérisation radicalaire de
a) 20 à 40% en poids d'amide de l'acide méthacrylique,
b) 40 à 70% en poids de vinylpyrrolidone,
en présence d'1 à 20% en poids de polymères d2) et/ou d'amidon et de dérivés d'amidon d3).

8. Copolymère A) tel que défini dans l'une des revendications 1 à 3, qui peut être obtenu par polymérisation radicalaire de
a) 30 à 40% en poids d'amide de l'acide méthacrylique,
b) 20 à 60% en poids de vinylpyrrolidone et 1 à 20% en poids de vinylcaprolactame.

9. Copolymère A) tel que défini dans l'une des revendications 1 à 3, qui peut être obtenu par copolymérisation radicalaire de
a) 5 à 50% en poids, par rapport au poids total des constituants a) à d), d'amide de l'acide méthacrylique,
b) 40 à 85% en poids, par rapport au poids total des constituants a) à d), d'au moins un composé, sélectionné parmi la vinylpyrrolidone, le vinylcaprolactame, le N,N-diméthylacrylamide et leurs mélanges,
c) 0,2 à 20% en poids, par rapport au poids total des constituants a) à d), d'au moins un composé différent de a) et b) et donc copolymérisable, insaturé et soluble dans l'eau, qui est sélectionné parmi le vinylimidazol et ses dérivés, les acrylates de polyéther et leurs mélanges,
éventuellement en présence de 10% en poids maximum, par rapport au poids total des constituants a) à d), de polymères d2) qui découlent de l'alcool vinylique, et éventuellement en présence d'1% en poids maximum, par rapport au poids total des constituants a) à d), d'au moins un réticulant.

10. Copolymère A) tel que défini dans l'une des revendications 1 à 3, qui peut être obtenu par polymérisation radicalaire de
a) 7 à 45% en poids d'amide de l'acide méthacrylique,
b) 50 à 80% en poids d'au moins un composé sélectionné parmi la vinylpyrrolidone, le vinylcaprolactame, le N,N-diméthylacrylamide et leurs mélanges,
c) 0,3 à 10% en poids d'au moins un composé sélectionné parmi le vinylimidazol et ses dérivés, les acrylates de polyéther et leurs mélanges,
en présence de 0,1 à 10% en poids de polymères d2) qui sont dérivés de l'alcool vinylique.

11. Copolymère A) tel que défini dans l'une des revendications 1 à 3, qui peut être obtenu par polymérisation radicalaire de
a) 10 à 45% en poids d'amide de l'acide méthacrylique,
b) 50 à 80% en poids de vinylpyrrolidone et de vinylcaprolactame et
c) 0,3 à 10% en poids de vinylmidazol et/ou d'un de ses dérivés.

12. Copolymère A) suivant la revendication 9, qui peut être obtenu par polymérisation radicalaire de
a) 10 à 45% en poids d'amide de l'acide méthacrylique,
b) 50 à 80% en poids de vinylpyrrolidone et
c) 0,5 à 5% en poids de vinylimidazol.

13. Procédé pour la préparation d'un copolymère A, tel que défini dans l'une des revendications 1 à 3 et 7 à 12, par polymérisation radicalaire des monomères a) avec au moins un autre monomère sélectionné parmi les monomères b) et c), éventuellement en présence de 25% en poids maximum, par rapport au poids total des constituants a) à d), d'un constituant soluble dans l'eau d), **caractérisé en ce que** l'on exécute la polymérisation dans un solvant aqueux.

14. Procédé suivant la revendication 13, **caractérisé en ce que** l'on exécute la polymérisation à un pH dans une plage comprise entre 6 et 8.

15. Procédé suivant la revendication 14, **caractérisé en ce que** l'on exécute la polymérisation à un pH dans une plage comprise entre 6,4 et 7,4.

16. Utilisation d'un copolymère A), tel que défini dans l'une des revendications 1 à 3 et 7 à 12, dans des produits de nettoyage de la peau, des agents pour l'entretien et la protection de la peau, des agents pour l'entretien des ongles, des préparations pour la cosmétique décorative et des agents de traitement capillaire.

17. Utilisation selon la revendication 16 dans des agents de traitement capillaire tels que des fixateurs et/ou des après-shampooings.

18. Utilisation selon la revendication 17, l'agent étant présent sous la forme d'un gel capillaire, d'un shampooing, d'une mousse fixante, d'une eau capillaire, d'un spray capillaire ou d'une mousse capillaire.

19. Utilisation d'un copolymère A), tel que défini dans l'une des revendications 1 à 3 et 7 à 12, comme auxiliaire dans l'industrie pharmaceutique, préférentiellement comme ou dans un(des) agent(s) d'enduction pour des formes de médicaments solides, ainsi que comme ou dans un(des) agent(s) d'enduction pour l'industrie textile, l'industrie du papier, l'imprimerie et l'industrie du cuir.

20. Utilisation d'un copolymère soluble dans l'eau A), tel que défini dans l'une des revendications 1 à 3 et 7 à 12, comme base de greffage, émulsifiant polymère ou colloïde de protection.
